# EUROPÄISCHE PATENTSCHRIFT

(11) **EP 2 528 522 B1**
(45) Veröffentlichungstag und Bekanntmachung des Hinweises auf die Patenterteilung: **08.05.2019**
(21) Anmeldenummer: 10798318.1
(22) Anmeldetag: 21.12.2010
(51) Int. Cl.: A61B 17/70

(54) **IMPLANTAT ZUR GEGENSEITIGEN ABSTÜTZUNG DER DORNFORTSÄTZE BENACHBARTER WIRBELKÖRPER SOWIE CHIRURGISCHES SYSTEM**
IMPLANT MUTUALLY SUPPORTING THE SPINOUS PROCESSES OF ADJACENT VERTEBRAL BODIES AND SURGICAL SYSTEM
IMPLANT POUR LE SOUTIEN RÉCIPROQUE DES APOPHYSES ÉPINEUSES DE CORPS VERTÉBRAUX ADJACENTS ET SYSTÈME CHIRURGICAL

(30) Priorität: 27.01.2010 DE 102010000231
(43) Veröffentlichungstag der Anmeldung: 05.12.2012
(73) Patentinhaber: Aesculap AG, 78532 Tuttlingen (DE)
(72) Erfinder: HAAS, Alexander, 78166 Donaueschingen (DE)
(74) Vertreter: Hoeger, Stellrecht & Partner Patentanwälte mbB
(86) Internationale Anmeldenummer: PCT/EP2010/070360
(87) Internationale Veröffentlichungsnummer: WO 2011/091917

(56) Entgegenhaltungen:
- DE-U1-202008 009 344
- DE-U1-202009 001 321

## Beschreibung

Die Erfindung betrifft ein Implantat zur gegenseitigen Abstützung der Dornfortsätze benachbarter Wirbelkörper, mit einer ersten Implantatkomponente und einer zweiten Implantatkomponente, wobei das Implantat von einer Einbringstellung, in der das Implantat zwischen einen oberen Dornfortsatz eines oberen Wirbelkörpers und einen unteren Dornfortsatz eines unteren Wirbelkörpers einbringbar ist, durch Bewegen der zweiten Implantatkomponente relativ zur ersten Implantatkomponente entlang einer Spreizrichtung in eine Spreizstellung überführbar ist, in der eine vom Implantat gebildete obere Stützfläche für den oberen Dornfortsatz und eine vom Implantat gebildete untere Stützfläche für den unteren Dornfortsatz einen größeren Abstand relativ zueinander einnehmen als in der Einbringstellung, und mit einer ein Verriegelungselement umfassenden Verriegelungseinrichtung, wobei das Verriegelungselement von einer Freigabestellung, in der die zweite Implantatkomponente entgegen der Spreizrichtung relativ zur ersten Implantatkomponente beweglich ist, relativ zu mindestens einer Implantatkomponente entlang einer quer zur Spreizrichtung ausgerichteten Verriegelungsrichtung in eine Verriegelungsstellung überführbar ist, in der die zweite Implantatkomponente gegen eine der Spreizrichtung entgegen gerichtete Bewegung an der ersten Implantatkomponente verriegelt ist, sowie mit einer Sicherungseinrichtung zur Sicherung des Verriegelungselementes gegen eine der Verriegelungsrichtung entgegen gerichtete Bewegung.

Außerdem betrifft die Erfindung ein chirurgisches System mit mindestens einem derartigen Implantat.

Ein Implantat der eingangs genannten Art ist in der DE 20 2008 009 344 U1 beschrieben. Es kann in der Einbringstellung in den Zwischenraum zwischen die Dornfortsätze eingebracht werden und anschließend in die Spreizstellung überführt werden. Dadurch vergrößern die Stützflächen ihren Abstand voneinander, so dass die auf den Stützflächen ruhenden Dornfortsätze mehr oder weniger auseinander geschoben werden können, zur Stabilisierung der benachbarten Wirbelkörper relativ zueinander. Zur Verriegelung des Implantates, d.h. zur Verriegelung der beiden Implantatkomponenten relativ zueinander, dient die Verriegelungseinrichtung, deren Verriegelungselement in der Spreizstellung des Implantates von der Freigabestellung in die Verriegelungsstellung überführt wird. Dabei kann das Verriegelungselement mit mindestens einem weiteren an der ersten Implantatkomponente angeordneten Verriegelungselement zusammenwirken, um eine Bewegung der zweiten Implantatkomponente entgegen der Spreizrichtung zu verhindern, so dass die zweite Implantatkomponente an der ersten Implantatkomponente verriegelt ist. Um zu verhindern, dass das Verriegelungselement sich entgegen der Verriegelungsrichtung aus der Verriegelungsstellung bewegt, weist das Implantat eine Sicherungseinrichtung auf. Im Falle des in der DE 20 2008 009 344 U1 beschriebenen Implantates ist dies ein zusätzliches Bauteil in Form einer das Verriegelungselement und die zweite Implantatkomponente teilweise übergreifende Kappe. Diese muss von einem Operateur nach dem Spreizen und Verriegeln des Implantates manuell angebracht werden, damit das Verriegelungselement in der Verriegelungsstellung gesichert ist.

Die DE 20 2009 001 321 U1 beschreibt ein chirurgisches Trenninstrument mit zwei über gegeneinander bewegbare Handgriffe aus einer Offenstellung in eine Schneidstellung bewegbaren Schneidbacken, wobei neben den Schneidbacken zwei aus einer Ausgangsstellung in eine Klemmstellung gegeneinander bewegbare Klemmbacken angeordnet sind und wobei an dem chirurgischen Trenninstrument Mitnehmer vorgesehen sind, die die Klemmbacken bei der Bewegung der Schneidbacken aus der Offenstellung in die Schneidstellung gleichzeitig von deren Ausgangsstellung in die Klemmstellung bewegen. Mit einem derartigen Trenninstrument können bei einem gattungsgemäßen, ebenfalls in der DE 20 2009 001 321 U1 beschriebenen Implantat vorgesehene Spannelemente abgetrennt werden und gleichzeitig eine Sicherungseinrichtung des Implantates betätigt werden. Die Sicherungseinrichtung umfasst an einem Verriegelungselement angeordnete Vorsprünge, die von Verformungsvorsprüngen der Schneidbacken erfasst und so umgebogen werden können, dass die Vorsprünge des die Verriegelungsstellung einnehmenden Verriegelungselementes in Eingriff mit einem die Implantatkomponenten zusammenhaltenden Fixierelement gebracht werden können. Das Abtrennen der Spannelemente und Sichern des Verriegelungselementes erfolgt dabei in einem Arbeitsschritt. Aufgabe der vorliegenden Erfindung ist es, ein gattungsgemäßes Implantat sowie ein gattungsgemäßes chirurgisches System jeweils so weiterzubilden, dass eine einfachere Sicherung des Verriegelungselementes gegen eine der Verriegelungsrichtung entgegen gerichtete Bewegung ermöglicht wird.

Diese Aufgabe wird bei einem Implantat der eingangs genannten Art erfindungsgemäß dadurch gelöst, dass die Sicherungseinrichtung mindestens ein erstes Sicherungsglied aufweist sowie mindestens ein zweites Sicherungsglied, die nach dem Einnehmen der Verriegelungsstellung durch das Verriegelungselement relativ zueinander in einer quer zur Verriegelungsrichtung ausgerichteten Sicherungsrichtung von einer entsicherten Stellung, in der das mindestens eine erste Sicherungsglied und das mindestens eine zweite Sicherungsglied nicht zusammenwirken, in eine gesicherte Stellung überführbar sind, in der das mindestens eine erste Sicherungsglied und das mindestens eine zweite Sicherungsglied zur Sicherung des Verriegelungselementes zusammenwirken, dass das Verriegelungselement das mindestens eine erste Sicherungsglied umfasst und eine Implantatkomponente das mindestens eine zweite Sicherungsglied umfasst, und dass das mindestens eine erste Sicherungsglied und das mindestens eine zweite Sicherungsglied in der entsicherten Stellung relativ zueinander in der Sicherungsrichtung vorgespannt sind.

Vorteilhafte Weiterbildungen der Erfindung sind Gegenstand der abhängigen Ansprüche.

Beim erfindungsgemäßen Implantat kann, anders als bei einem herkömmlichen Implantat, eine separate Sicherungseinrichtung entfallen, weil das Verriegelungselement das mindestens eine erste Sicherungsglied und eine Implantatkomponente das mindestens eine zweite Sicherungsglied umfasst. Somit kann das Verriegelungselement, um gesichert zu werden, unmittelbar mit der Implantatkomponente zusammenwirken. Dies erfolgt mittels der Sicherungsglieder in der gesicherten Stellung. Damit übt das Verriegelungselement über die Verriegelung der beiden Implantatkomponenten relativ zueinander hinaus eine weitere Funktion aus, nämlich die Eigensicherung an einer Implantatkomponente. Dies vereinfacht den Aufbau des Implantates.

Das mindestens eine erste Sicherungsglied und das mindestens eine zweite Sicherungsglied sind beim erfindungsgemäßen Implantat relativ zueinander beweglich ausgebildet. Nach dem Einnehmen der Verriegelungsstellung durch das Verriegelungselement können sie von einer entsicherten Stellung in eine gesicherte Stellung überführt werden. In der gesicherten Stellung kann das Verriegelungselement gegen eine der Verriegelungsrichtung entgegen gerichtete Bewegung gesichert werden. Das mindestens eine erste Sicherungsglied und das mindestens eine zweite Sicherungsglied sind relativ zueinander zum Überführen von der entsicherten Stellung in die gesicherte Stellung entlang einer Sicherungsrichtung beweglich ausgebildet, die quer zur Verschieberichtung ausgerichtet ist. Wirkt in der Sicherungsrichtung eine Kraft, führt dies dazu, dass das mindestens eine erste und das mindestens eine zweite Sicherungsglied relativ zueinander gewissermaßen "automatisch" von der entsicherten Stellung in die gesicherte Stellung überführt werden. Dies vereinfacht das Sichern des Verriegelungselementes in der Verriegelungsstellung, und es muss insbesondere von einem Operateur nicht in einem zusätzlichen Arbeitsgang nach dem Verriegeln des Implantates durchgeführt werden. Eine in der Sicherungsrichtung wirksame Kraft kann sich beispielsweise dadurch ergeben, dass die Dornfortsätze auf die Stützflächen des Implantates eine der vom Implantat ausgeübten Spreizkraft entgegen gerichtete Stützkraft einleiten, welche entlang der Spreizrichtung abgeleitet werden kann und entlang der Sicherungsrichtung umgelenkt wird.

Die Verriegelungsrichtung ist quer und insbesondere senkrecht zur Spreizrichtung ausgerichtet, denn dies hat sich in der Praxis als günstig für die Verriegelung des Implantates und die Konstruktion desselben erwiesen.

Das mindestens eine erste Sicherungsglied und das mindestens eine zweite Sicherungsglied sind in der entsicherten Stellung relativ zueinander entlang der Sicherungsrichtung vorgespannt. Durch die Vorspannung ist die Möglichkeit gegeben, dass das mindestens eine erste Sicherungsglied und das mindestens eine zweite Sicherungsglied gewissermaßen "automatisch" von der entsicherten Stellung in die gesicherte Stellung überführt werden. Dies führt dazu, dass dann, wenn das Verriegelungselement die Verriegelungsstellung einnimmt, automatisch eine Sicherung des Verriegelungselementes in der Verriegelungsstellung erfolgen kann. Dies erleichtert die Handhabung des Implantates erheblich. Einem Operateur bleibt es insbesondere erspart, nach dem Verriegeln des Implantates zusätzlich die Sicherung des Verriegelungselementes vorzunehmen.

Zur Vorspannung des mindestens einen ersten Sicherungsgliedes und des mindestens einen zweiten Sicherungsgliedes relativ zueinander kann beispielsweise mindestens ein auf ein Sicherungsglied einwirkendes elastisches Element, wie etwa eine Feder, vorgesehen sein. Das elastische Element kann z.B. mit einem ersten Ende am Verriegelungselement und mit einem zweiten Ende an einem Sicherungsglied angreifen, und/oder es kann z.B. mit einem ersten Ende an der Implantatkomponente und mit einem zweiten Ende an einem Sicherungsglied angreifen.

Optional kann bei einem Implantat der eingangs genannten Art, bei welchem die Sicherungseinrichtung mindestens ein erstes Sicherungsglied aufweist sowie mindestens ein zweites Sicherungsglied, die nach dem Einnehmen der Verriegelungsstellung durch das Verriegelungselement relativ zueinander in einer quer zur Verriegelungsrichtung ausgerichteten Sicherungsrichtung von einer entsicherten Stellung, in der das mindestens eine erste Sicherungsglied und das mindestens eine zweite Sicherungsglied nicht zusammenwirken, in eine gesicherte Stellung überführbar sind, in der das mindestens eine erste Sicherungsglied und das mindestens eine zweite Sicherungsglied zur Sicherung des Verriegelungselementes zusammenwirken, vorgesehen sein, dass das mindestens eine erste Sicherungsglied und das mindestens eine zweite Sicherungsglied in der entsicherten Stellung relativ zueinander entlang der Sicherungsrichtung vorgespannt sind. Auf diese Weise kann der vorstehend beschriebene Vorteil der gewissermaßen automatischen Sicherung des Verriegelungselementes in der Verriegelungsstellung ebenfalls erzielt werden. Von der zuletzt beschriebenen vorteilhaften Ausführungsform des erfindungsgemäßen Implantates unterscheidet sich dieses Implantat dadurch, dass das Verriegelungselement nicht notwendigerweise das mindestens eine erste Sicherungsglied umfasst und dass eine Implantatkomponente nicht notwendigerweise das mindestens eine zweite Sicherungsglied umfasst.

Günstig ist es, wenn mindestens ein Sicherungsglied und bevorzugt alle Sicherungsglieder einen entgegen der Verriegelungsrichtung wirksamen Anschlag umfasst oder ausbildet. Dies ermöglicht eine einfache Konstruktion des Implantates. Die jeweiligen Anschläge zweier Sicherungsglieder, und zwar eines ersten Sicherungsgliedes am Verriegelungselement und eines zweiten Sicherungsgliedes an der Implantatkomponente, können in der gesicherten Stellung der Sicherungsglieder zusammenwirken.

Von Vorteil ist es, wenn mindestens ein Sicherungsglied als Vorsprung eines es umfassenden Trägers ausgebildet ist, denn auch dies ermöglicht eine einfache Konstruktion des Implantates. Bei dem Träger handelt es sich beispielsweise um das Verriegelungselement oder um die Implantatkomponente und insbesondere um ein Fixierelement der Implantatkomponente, welches in der Verriegelungsstellung des Verriegelungselementes mit diesem zur Verriegelung des Implantates zusammenwirkt. Der Vorsprung umfasst günstigerweise mindestens einen entgegen der Verriegelungsrichtung wirksamen Anschlag.

Vorzugsweise ist mindestens ein Sicherungsglied eine an einem es umfassenden Träger gebildete Aufnahme, denn auch dies ermöglicht eine einfache Konstruktion des Implantates. Bei dem Träger handelt es sich ebenfalls beispielsweise um das Verriegelungselement, die Implantatkomponente oder insbesondere das davon umfasste Fixierelement. Die Aufnahme umfasst günstigerweise mindestens einen entgegen der Verriegelungsrichtung wirksamen Anschlag.

Von Vorteil ist es, wenn das mindestens eine erste Sicherungsglied und das mindestens eine zweite Sicherungsglied in der gesicherten Stellung aneinander anliegen. Dies ermöglicht eine wirkungsvolle Sicherung des Verriegelungselementes in der Verriegelungsstellung. Auf diese Weise kann beispielsweise ein Formschluss zwischen der Implantatkomponente und dem Verriegelungselement in dessen Verriegelungsstellung entgegen der Verriegelungsrichtung gewährleistet werden.

In der entsicherten Stellung können das mindestens eine erste Sicherungsglied und das mindestens eine zweite Sicherungsglied und/oder von diesen jeweils umfasste oder ausgebildete Anschläge beispielsweise einen Abstand voneinander aufweisen, um nicht zusammenzuwirken.

Bevorzugt greifen das mindestens eine erste Sicherungsglied und das mindestens eine zweite Sicherungsglied in der gesicherten Stellung ineinander ein, denn dies ermöglicht eine zuverlässige Sicherung des Verriegelungselementes und zugleich eine einfache Konstruktion des Implantates. Beispielsweise greift ein als Vorsprung gebildetes Sicherungsglied in ein als Aufnahme gebildetes Sicherungsglied ein.

In entsprechender Weise erfolgt eine zuverlässige Sicherung des Verriegelungselementes bei einer einfachen Konstruktion des Implantates günstigerweise dadurch, dass das mindestens eine erste Sicherungsglied und das mindestens eine zweite Sicherungsglied einander in der gesicherten Stellung hintergreifen. Dabei liegen von den Sicherungsgliedern umfasste oder ausgebildete Anschläge günstigerweise aneinander an.

Vorzugsweise ist mindestens ein Sicherungsglied endseitig am Verriegelungselement angeordnet, bezogen auf dessen Erstreckung entlang der Verriegelungsrichtung. Es hat sich in der Praxis gezeigt, dass dadurch dem Implantat eine einfachere Konstruktion verliehen werden kann. Insbesondere kann das Verriegelungselement zwei an seinen einander gegenüberliegenden Enden angeordnete erste Sicherungsglieder umfassen.

Von Vorteil ist es, wenn mindestens ein Sicherungsglied und bevorzugt alle ersten Sicherungsglieder einstückig mit dem Verriegelungselement ausgebildet sind, denn dies ermöglicht eine einfachere Konstruktion des Implantates.

Bei dem erfindungsgemäßen Implantat umfasst eine Implantatkomponente das mindestens eine zweite Sicherungsglied. Bei einer bevorzugten Ausführungsform des Implantates kann vorgesehen sein, dass die zweite Implantatkomponente das mindestens eine zweite Sicherungsglied oder mindestens ein zweites Sicherungsglied umfasst.

Alternativ oder ergänzend kann vorgesehen sein, dass die erste Implantatkomponente mindestens ein zweites Sicherungsglied umfasst.

Von Vorteil ist es, wenn die zweite Implantatkomponente ein mit dem Verriegelungselement zum Verriegeln des Implantates zusammenwirkendes Fixierelement aufweist, das das mindestens eine zweite Sicherungsglied umfasst. Das Fixierelement kann mit dem Verriegelungselement in dessen Verriegelungsstellung zusammenwirken, so dass die zweite Implantatkomponente gegen eine der Spreizrichtung entgegen gerichtete Bewegung an der ersten Implantatkomponente verriegelt ist. Zugleich umfasst es das mindestens eine zweite Sicherungsglied, das in der gesicherten Stellung mit dem vom Verriegelungselement umfassten mindestens einen ersten Sicherungsglied zur Sicherung des Verriegelungselementes zusammenwirkt. Dadurch kann das Fixierelement ebenso wie das Verriegelungselement in zweifacher Hinsicht wirksam sein, nämlich zum einen zum Verriegeln des Implantates und zum anderen zum Sichern des Verriegelungselementes. Dies erlaubt es, dem Implantat eine einfachere Konstruktion zu verleihen.

Günstig ist es, wenn das Fixierelement ein am Verriegelungselement in dessen Verriegelungsstellung anliegendes Anlageelement ausbildet. Die zweite Implantatkomponente kann dadurch zum Verriegeln des Implantates am Verriegelungselement, welches ebenfalls ein Anlageelement ausbildet, anliegen. Dies erlaubt es, die zweite Implantatkomponente auf konstruktiv einfache Weise an der ersten Implantatkomponente zu verriegeln. Die Anlageelemente bilden hierfür entgegen der Spreizrichtung zusammenwirkende wirksame Anschläge.

Vorzugsweise ist mindestens ein Sicherungsglied endseitig am Fixierelement angeordnet, bezogen auf dessen Erstreckung entlang der Verriegelungsrichtung. Es hat sich in der Praxis gezeigt, dass dadurch eine einfachere Konstruktion des Implantates möglich ist. Insbesondere kann das Fixierelement zwei zweite Sicherungsglieder umfassen, die an seinen einander gegenüberliegenden Enden angeordnet sind.

Bevorzugt ist mindestens ein Sicherungsglied und sind insbesondere alle zweiten Sicherungsglieder einstückig mit dem Fixierelement ausgebildet. Dies ermöglicht eine einfachere Konstruktion des Implantates.

Günstig ist es, wenn das Verriegelungselement plattenförmig oder im Wesentlichen plattenförmig ausgebildet ist, denn auch dies vereinfacht die Konstruktion des Implantates. Ein plattenförmiges oder im Wesentlichen plattenförmiges Verriegelungselement kann wirkungsvoll ein Anlageelement ausbilden, über welches das Verriegelungselement flächig am Fixierelement anliegen kann. Dies gibt die Möglichkeit einer zuverlässigen Verriegelung des Implantates. Besonders bevorzugt ist es beispielsweise, wenn das Verriegelungselement einen plattenförmigen Träger und einen von diesem abstehenden Vorsprung umfasst, der ein Sicherungsglied ausbildet.

Günstig ist es, wenn mindestens ein Sicherungsglied und bevorzugt alle Sicherungsglieder und/oder das Verriegelungselement und/oder ein mit dem Verriegelungselement zum Verriegeln des Implantates zusammenwirkendes Fixierelement der zweiten Implantatkomponente starr ausgebildet sind. Dadurch kann eine zuverlässige Verriegelung des Implantates gewährleistet werden und/oder eine zuverlässige Sicherung des Verriegelungselementes in der Verriegelungsstellung.

Vorzugsweise umfassen das Verriegelungselement und die das mindestens eine zweite Sicherungsglied umfassende Implantatkomponente jeweils zwei in der gesicherten Stellung paarweise zusammenwirkende Sicherungsglieder, d.h. das Verriegelungselement umfasst zwei erste Sicherungsglieder, und die Implantatkomponente umfasst zwei zweite Sicherungsglieder. Dadurch kann eine zuverlässige Sicherung des Verriegelungselementes sichergestellt werden.

Es hat sich zur Erzielung einer einfachen Konstruktion des Implantates und einer zuverlässigen Verriegelung desselben als vorteilhaft erwiesen, wenn das Verriegelungselement relativ zur ersten Implantatkomponente und/oder relativ zur zweiten Implantatkomponente zur Überführung von der Freigabestellung in die Verriegelungsstellung verschieblich ausgebildet ist.

Die Sicherungsrichtung ist vorzugsweise senkrecht zur Verriegelungsrichtung ausgerichtet. Dadurch weist sie keine parallel zur Verriegelungsrichtung ausgerichtete Komponente auf. Dies gibt die Möglichkeit, dass das mindestens eine Sicherungsglied und das mindestens eine zweite Sicherungsglied beim Überführen von der entsicherten Stellung in die gesicherte Stellung eine maximal kurze Relativbewegung zueinander ausführen müssen, damit das Verriegelungselement in der Verriegelungsstellung gesichert werden kann. Dies ermöglicht zum einen eine einfachere Konstruktion des Implantates und zum anderen eine zuverlässige Sicherung des Verriegelungselementes.

Von Vorteil ist es, wenn die zweite Implantatkomponente relativ zur ersten Implantatkomponente entgegen der Spreizrichtung in der Verriegelungsstellung des Verriegelungselementes vorgespannt ist. Dadurch wirkt auf die Implantatkomponenten eine Vorspannkraft ein, die sie zu einer Bewegung relativ zueinander entgegen der Spreizrichtung beaufschlagt. Wenn das Verriegelungselement in der Verriegelungsstellung zum Verriegeln des Implantates an der ersten Implantatkomponente angreift und die zweite Implantatkomponente das mindestens eine zweite Sicherungsglied umfasst, kann die Vorspannkraft dazu ausgenutzt werden, eine Vorspannung zwischen dem mindestens einen ersten Sicherungsglied und dem mindestens einen zweiten Sicherungsglied zu gewährleisten. Dadurch lässt sich die dem Implantat innewohnende Vorspannung der beiden Implantatkomponenten relativ zueinander zur automatischen Überführung der Sicherungsglieder von der entsicherten Stellung in die gesicherte Stellung ausnutzen. Dies erleichtert die Handhabung des Implantates, und es ermöglicht zudem eine einfachere Konstruktion desselben.

Eine Vorspannung des mindestens einen ersten Sicherungsgliedes und des mindestens einen zweiten Sicherungsgliedes relativ zueinander in der entsicherten Stellung kann auch dadurch erzielt werden, dass über die Stützflächen eine von den Dornfortsätzen ausgeübte Stützkraft auf die beiden Implantatkomponenten eingeleitet und entlang der Spreizrichtung abgeleitet wird. Diese Stützkraft kann wie die vorstehend beschriebene Vorspannkraft wirken, so dass dann, wenn das Verriegelungselement in der Verriegelungsstellung an der ersten Implantatkomponente angreift und die zweite Implantatkomponente das mindestens eine zweite Sicherungsglied umfasst, in der entsicherten Stellung eine Vorspannung der Sicherungsglieder relativ zueinander erzielt werden kann und damit auch eine automatische Sicherung des Verriegelungselements, wenn es die Verriegelungsstellung einnimmt.

Bevorzugt weist die Sicherungsrichtung eine zur Spreizrichtung parallele Komponente auf, und günstigerweise ist die Sicherungsrichtung parallel zur Spreizrichtung ausgerichtet. Die vorstehend beschriebene Vorspannkraft bzw. Stützkraft entlang der Spreizrichtung kann auf diese Weise unmittelbar, d.h. ohne umgelenkt zu werden, zur Vorspannung des mindestens einen ersten Sicherungsgliedes und des mindestens einen zweiten Sicherungsgliedes relativ zueinander führen. Eine Einrichtung zur Umlenkung der Vorspannkraft bzw. der Stützkraft kann dadurch entfallen. Dies vereinfacht die Konstruktion des Implantates.

Von Vorteil ist es, wenn ein mit dem Verriegelungselement zum Verriegeln des Implantates zusammenwirkendes Fixierelement der zweiten Implantatkomponente und das Verriegelungselement jeweils mindestens ein Führungsglied ausbilden oder ein Führungsglied umfassen, das beim Überführen des Verriegelungselementes von der Freigabestellung in die Verriegelungsstellung mit einem weiteren vom Verriegelungselement bzw. vom Fixierelement ausgebildeten oder vom Verriegelungselement bzw. vom Fixierelement umfassten Führungsglied zusammenwirkt. Dadurch kann das Verriegelungselement auf zuverlässige Weise in einer klar definierten Verriegelungsrichtung von der Freigabestellung in die Verriegelungsstellung überführt werden.

Günstig ist es, wenn mindestens ein Sicherungsglied ein Führungsglied ausbildet, denn dies ermöglicht eine einfachere Konstruktion des Implantates.

Von Vorteil ist es, wenn das Verriegelungselement mindestens ein erstes Angriffsglied umfasst oder ausbildet zur Beaufschlagung des Verriegelungselementes durch eine in Spreizrichtung gerichtete Spreizkraft und wenn das Verriegelungselement mindestens ein zweites Angriffsglied umfasst oder ausbildet zur Anlage an eine Implantatkomponente beim Überführen des Implantates von der Einbringstellung in die Spreizstellung. Dies gibt die Möglichkeit, das Verriegelungselement als Lagerelement zur Überführung des Implantates von der Einbringstellung in die Spreizstellung einzusetzen. Das Verriegelungselement kann an dem mindestens einen ersten Angriffsglied mit einer Spreizkraft beaufschlagt werden, und es kann seinerseits die Spreizkraft mittels des mindestens einen zweiten Angriffsgliedes an die Implantatkomponente übertragen. Beispielsweise kann das Verriegelungselement die Spreizkraft an das bereits erwähnte von der zweiten Implantatkomponente umfasste Fixierelement übertragen.

Vorzugsweise bildet mindestens ein Sicherungsglied ein Angriffsglied aus, denn dies ermöglicht eine einfachere Konstruktion des Implantates.

Wie eingangs erwähnt, betrifft die Erfindung auch ein chirurgisches System mit mindestens einem Implantat der eingangs genannten Art. Bei einem erfindungsgemäßen chirurgischen System wird die vorstehend gestellte Aufgabe erfindungsgemäß dadurch gelöst, dass das Implantat als erfindungsgemäßes Implantat ausgebildet ist und dass das chirurgische System eine Handhabungsvorrichtung zum Überführen des Verriegelungselementes von der Freigabestellung in die Verriegelungsstellung umfasst.

Durch die Ausbildung des mindestens einen Implantates als erfindungsgemäßes Implantat können die mit diesem erzielbaren Vorteile ebenfalls erzielt werden. Diese Vorteile wurden bereits im Zusammenhang mit der Erläuterung des erfindungsgemäßen Implantates erwähnt.

Darüber hinaus umfasst das chirurgische System eine Handhabungsvorrichtung zum Überführen des Verriegelungselementes von der Freigabestellung in die Verriegelungsstellung. Dieser Handhabungsvorrichtung kann sich ein Operateur bedienen, um das Implantat zu verriegeln. Dies erleichtert die Verriegelung des Implantates und erhöht zugleich die Benutzerfreundlichkeit des chirurgischen Systems.

Das chirurgische System kann insbesondere eines der vorstehend beschriebenen Implantate umfassen.

Günstig ist es, wenn das System eine Kopplungseinrichtung aufweist mit mindestens einem vom Verriegelungselement umfassten ersten Kopplungsglied und mit mindestens einem von der Handhabungsvorrichtung umfassten zweiten Kopplungsglied, die zum Überführen des Verriegelungselementes von der Freigabestellung in die Verriegelungsstellung zusammenwirken. Die Kopplungseinrichtung ermöglicht es, mittels der Handhabungsvorrichtung das Verriegelungselement von der Freigabestellung in die Verriegelungsstellung zu überführen.

Von Vorteil ist es, wenn mindestens ein Kopplungsglied und bevorzugt alle Kopplungsglieder einen in der Verriegelungsrichtung wirksamen Anschlag umfasst oder ausbildet, denn dies ermöglicht eine einfache Konstruktion der Kopplungseinrichtung. Der Anschlag ist insbesondere ein Mitnehmeranschlag, durch dessen Wirkung das mindestens eine zweite Kopplungsglied das mindestens eine erste Kopplungsglied mit einer in der Verriegelungsrichtung gerichteten Kraft beaufschlagt, um das Verriegelungselement von der Freigabestellung in die Verriegelungsstellung zu überführen.

Vorteilhafterweise ist mindestens ein Kopplungsglied als Vorsprung eines es umfassenden Trägers ausgebildet. Bei dem Träger kann es sich beispielsweise um das Verriegelungselement handeln oder um die Handhabungsvorrichtung. Der Vorsprung umfasst günstigerweise den Anschlag oder bildet diesen aus.

Vorzugsweise ist mindestens ein Kopplungsglied eine an einem es umfassenden Träger gebildete Aufnahme. Der Träger ist beispielsweise das Verriegelungselement oder die Handhabungsvorrichtung. Die Aufnahme bildet günstigerweise den Anschlag aus oder umfasst denselben.

Günstig ist es, wenn das mindestens eine erste Kopplungsglied und das mindestens eine zweite Kopplungsglied zum Überführen des Verriegelungselementes von der Freigabestellung in die Verriegelungseinrichtung aneinander anliegen. Dadurch kann das Verriegelungselement auf zuverlässige Weise in die Verriegelungsstellung überführt werden. Beispielsweise liegen die Kopplungsglieder mit jeweils von ihnen gebildeten oder umfassten Anschlägen und insbesondere Mitnehmeranschlägen aneinander an.

Es kann vorgesehen sein, dass das mindestens eine erste Kopplungsglied und das mindestens eine zweite Kopplungsglied zum Überführen des Verriegelungselementes von der Freigabestellung in die Verriegelungsstellung ineinander eingreifen. Dies kann z.B. dadurch erfolgen, dass ein Kopplungsglied einen Vorsprung ausbildet und ein weiteres Kopplungsglied eine Aufnahme ausbildet, in die der Vorsprung eingreift.

Ebenso kann vorgesehen sein, dass das mindestens eine erste Kopplungsglied und das mindestens eine zweite Kopplungsglied zum Überführen des Verriegelungselementes von der Freigabestellung in die Verriegelungsstellung einander hintergreifen. Dabei liegen günstigerweise von den Kopplungsgliedern jeweils ausgebildete oder umfasst Anschläge und insbesondere Mitnehmeranschläge aneinander an. Dies ermöglicht eine zuverlässige Überführung des Verriegelungselementes von der Freigabestellung in die Verriegelungsstellung.

Von Vorteil ist es, wenn das mindestens eine erste Kopplungsglied endseitig am Verriegelungselement angeordnet ist, bezogen auf dessen Erstreckung entlang der Verriegelungsrichtung. Dies hat sich in der Praxis als zuverlässig für eine wirkungsvolle Kopplung mit der Handhabungsvorrichtung erwiesen.

Eine einfachere Konstruktion des Implantates wird dadurch ermöglicht, dass das mindestens eine erste Kopplungsglied einstückig mit dem Verriegelungselement ausgebildet ist.

Günstig ist es, wenn das mindestens eine erste Kopplungsglied und das mindestens eine zweite Kopplungsglied nach dem Einnehmen der Verriegelungsstellung durch das Verriegelungselement relativ zueinander in einer quer zur Verriegelungsrichtung ausgerichteten Entkopplungsrichtung von einer Kopplungsstellung, in der das mindestens eine erste Kopplungsglied und das mindestens eine zweite Kopplungsglied zum Überführen des Verriegelungselementes von der Freigabestellung in die Verriegelungsstellung zusammenwirken, in eine Entkopplungsstellung überführbar sind, in der das mindestens eine erste Kopplungsglied und das mindestens eine zweite Kopplungsglied nicht zusammenwirken. In der Kopplungsstellung können das mindestens eine erste und das mindestens eine zweite Kopplungsglied zum Überführen des Verriegelungselementes in die Verriegelungsstellung zusammenwirken. Nimmt das Verriegelungselement die Verriegelungsstellung ein, haben die Kopplungsglieder ihren bestimmungsgemäßen Zweck erfüllt. Sie können voneinander entkoppeln, indem sie relativ zueinander entlang einer Entkopplungsrichtung in eine Entkopplungsstellung überführt werden, in welcher sie nicht mehr zusammenwirken. Dies gibt beispielsweise die Möglichkeit, die Handhabungsvorrichtung nach dem Verriegeln des Implantates von diesem abzuziehen oder zu entfernen, weil sie nicht weiter zur Verriegelung des Implantates benötigt wird.

In der Entkopplungsstellung können das mindestens eine erste Kopplungsglied und das mindestens eine zweite Kopplungsglied und/oder von diesen jeweils umfasste oder ausgebildete Anschläge beispielsweise einen Abstand voneinander aufweisen, um nicht zusammenzuwirken.

Vorzugsweise sind das mindestens eine erste Kopplungsglied und das mindestens eine zweite Kopplungsglied in der Kopplungsstellung entlang der Entkopplungsrichtung relativ zueinander vorgespannt. Dies erlaubt es, das mindestens eine erste Kopplungsglied und das mindestens eine zweite Kopplungsglied gewissermaßen "automatisch" voneinander zu entkoppeln, d.h. von der Kopplungsstellung in die Entkopplungsstellung zu überführen. Dies funktioniert in entsprechender zu der vorstehend erläuterten Weise für den Fall des mindestens einen ersten Sicherungsgliedes und des mindestens einen zweiten Sicherungsgliedes, die in Folge einer Vorspannung automatisch von der entsicherten in die gesicherte Stellung überführt werden können. Im vorliegenden Fall kann nach dem Einnehmen der Verriegelungsstellung durch das Verriegelungselement eine Entkopplung des mindestens einen ersten und des mindestens einen zweiten Kopplungsgliedes automatisch erzielt werden.

Eine Vorspannung des mindestens einen ersten Kopplungsgliedes und des mindestens einen zweiten Kopplungsgliedes relativ zueinander kann beispielsweise auf der Vorspannung der beiden Implantatkomponenten relativ zueinander beruhen. In entsprechender Weise kann eine von den Dornfortsätzen auf das Implantat eingeleitete und ebenfalls entgegen der Spreizrichtung wirkende Stützkraft demselben Zweck dienen. Damit ist insbesondere die Möglichkeit gegeben, dass die Vorspannkraft bzw. die Stützkraft jeweils sowohl zum automatischen Überführen der Sicherungsglieder von der entsicherten Stellung in die gesicherte Stellung wirksam werden können als auch zum automatischen Überführen der Kopplungsglieder von der Kopplungsstellung in die Entkopplungsstellung.

Bevorzugt weist die Entkopplungsrichtung eine zur Spreizrichtung parallele Komponente auf, und günstigerweise ist die Entkopplungsrichtung parallel zur Spreizrichtung ausgerichtet. Dies gibt die Möglichkeit, dass eine Vorspannkraft zwischen der ersten Implantatkomponente und der zweiten Implantatkomponente entgegen der Spreizrichtung in der Verriegelungsstellung des Verriegelungselementes, ohne umgelenkt werden zu müssen, zum Überführen des mindestens einen ersten Kopplungsgliedes und des mindestens einen zweiten Kopplungsgliedes von der Kopplungsstellung in die Entkopplungsstellung wirksam werden kann.

Damit eine Vorspannkraft bzw. eine Stützkraft zur Entkopplung der Kopplungsglieder eingesetzt werden kann, kann es erforderlich sein, dass die Handhabungsvorrichtung mit dem Implantat gekoppelt ist. Günstigerweise umfasst die Handhabungsvorrichtung mindestens ein Koppelelement zur Kopplung des mindestens einen zweiten Kopplungsgliedes mit einer Implantatkomponente in der Verriegelungsstellung des Verriegelungselementes. Eine Vorspannkraft bzw. Stützkraft, die auf die Implantatkomponente einwirkt, kann mittels des Koppelelementes auf die Handhabungsvorrichtung und insbesondere das mindestens eine zweite Kopplungsglied übertragen werden. Zugleich kann die Vorspannkraft bzw. die Stützkraft über die andere Implantatkomponente auf das Verriegelungselement und insbesondere das mindestens eine erste Kopplungsglied einwirken. Auf diese Weise können das mindestens eine erste Kopplungsglied und das mindestens eine zweite Kopplungsglied in der Kopplungsstellung relativ zueinander vorgespannt werden, und nach dem Einnehmen der Verriegelungsstellung durch das Verriegelungselement können sie automatisch in die Entkopplungsstellung überführt werden.

Günstig ist es, wenn das mindestens eine Koppelelement das mindestens eine zweite Kopplungsglied mit einem mit dem Verriegelungselement zum Verriegeln des Implantates zusammenwirkenden Fixierelement der zweiten Implantatkomponente koppelt. Dies gibt die Möglichkeit, eine Vorspannkraft bzw. eine Stützkraft über die zweite Implantatkomponente und insbesondere dessen Fixierelement auf das mindestens eine zweite Kopplungsglied zu übertragen.

Das mindestens eine Kopplungselement steht mit der Implantatkomponente und insbesondere dem Fixierelement der zweiten Implantatkomponente beispielsweise in kraft- und/oder formschlüssigem Eingriff. Mit dem mindestens einen zweiten Kopplungsglied kann es beispielsweise einstückig verbunden sein und dadurch die Kopplung erzielt werden.

Von Vorteil ist es, wenn die Handhabungsvorrichtung eine Aufnahme für das Verriegelungselement umfasst. Die Aufnahme kann zum Schutz und zur verbesserten Handhabung des Verriegelungselementes dienen. Dieses kann in der Aufnahme beispielsweise gehalten sein.

Bevorzugt umfasst die Aufnahme eine Öffnung, durch die hindurch das Verriegelungselement in der Kopplungsstellung des mindestens einen ersten Kopplungsgliedes und des mindestens einen zweiten Kopplungsgliedes nicht aus der Aufnahme entfernbar und durch die hindurch das Verriegelungselement in der Entkopplungsstellung des mindestens einen ersten Kopplungsgliedes und des mindestens einen zweiten Kopplungsgliedes in einer quer zur Entkopplungsrichtung ausgerichteten Richtung aus der Aufnahme entfernbar ist. In der Kopplungsstellung wirken die Kopplungsglieder zusammen, um das Verriegelungselement in die Verriegelungsstellung zu überführen. Währenddessen ist das Verriegelungselement in der Aufnahme angeordnet und kann aus dieser nicht durch die Öffnung hindurch entfernt werden. Nach dem Einnehmen der Verriegelungsstellung durch das Verriegelungselement können sich das mindestens eine erste Kopplungsglied und das mindestens eine zweite Kopplungsglied entlang der Entkopplungsrichtung in die Entkopplungsstellung bewegen. Das Implantat ist mittels des Verriegelungselementes verriegelt, und das Verriegelungselement kann durch die Öffnung hindurch aus der Aufnahme der Handhabungsvorrichtung entfernt werden. Dies ermöglicht beispielsweise das Abziehen der Handhabungsvorrichtung vom Implantat nach dessen Verriegelung, etwa entlang der quer zur Entkopplungsrichtung ausgerichteten Verriegelungsrichtung.

Die Öffnung der Aufnahme ist günstigerweise endseitig an der Handhabungsvorrichtung angeordnet, und zwar bevorzugt an dem Ende der Handhabungsvorrichtung, das demjenigen Ende der Handhabungsvorrichtung entgegengesetzt ist, an dem ein Operateur zum Abziehen der Handhabungsvorrichtung vom Implantat angreift.

Von Vorteil ist es, wenn das mindestens eine zweite Kopplungsglied zumindest teilweise einen Rand der Öffnung bildet oder daran angeordnet ist, denn dies ermöglicht eine einfachere konstruktive Ausgestaltung der Handhabungsvorrichtung. Das Verriegelungselement kann so lange nicht durch die Öffnung hindurch treten, wie die Kopplungsglieder die Kopplungsstellung einnehmen, wobei das mindestens eine erste Kopplungsglied an einem Rand der Öffnung anliegt. Nehmen die Kopplungsglieder die Entkopplungsstellung ein, wird gewissermaßen die Öffnung frei für das Verriegelungselement, und es kann durch die Öffnung hindurch treten und aus der Aufnahme entfernt werden.

Vorzugsweise sind das Verriegelungselement und ein mit dem Verriegelungselement zum Verriegeln des Implantates zusammenwirkendes Fixierelement der zweiten Implantatkomponente entlang der Entkopplungsrichtung in der Kopplungsstellung des mindestens einen ersten Kopplungsgliedes und des mindestens einen zweiten Kopplungsgliedes spielfrei und insbesondere formschlüssig in der Aufnahme angeordnet und in der Entkopplungsstellung des mindestens einen ersten Kopplungsgliedes und des mindestens zweiten Kopplungsgliedes mit Spiel in der Aufnahme angeordnet. Die Anordnung des Fixierelementes in der Aufnahme zusätzlich zum Verriegelungselement dient dazu, eine zuverlässige relative Positionierung des Fixierelementes und des Verriegelungselementes bei dessen Überführen von der Freigabestellung in die Verriegelungsstellung sicherzustellen. Außerdem ist dadurch das Implantat sicher an der Handhabungsvorrichtung halterbar. Entlang der Entkopplungsrichtung können das Verriegelungselement und das Fixierelement in der Kopplungsstellung der Kopplungsglieder einen größeren Abstand relativ zueinander einnehmen als in der Entkopplungsstellung. Die vorstehend beschriebene Öffnung der Aufnahme kann dabei entlang der Entkopplungsstellung insbesondere so dimensioniert sein, dass das Fixierelement und das Verriegelungselement in der Entkopplungsstellung der Kopplungsglieder durch die Öffnung aus der Aufnahme entfernbar sind, wohingegen sie in der Kopplungsstellung der Kopplungsglieder nicht aus der Aufnahme entfernbar sind. Beim Entkoppeln der Kopplungsglieder kann dadurch auf konstruktiv einfache Weise sichergestellt werden, dass das Verriegelungselement und das Fixierelement durch die Öffnung aus der Aufnahme entfernt werden können.

Von Vorteil ist es, wenn das Verriegelungselement und/oder ein mit dem Verriegelungselement zum Verriegeln des Implantates zusammenwirkendes Fixierelement der zweiten Implantatkomponente entlang der Verriegelungsrichtung beim Überführen des Verriegelungselementes von der Freigabestellung in die Verriegelungsstellung spielfrei und insbesondere formschlüssig in der Aufnahme angeordnet sind. Dies ermöglicht eine zuverlässigere Überführung des Verriegelungselementes von der Freigabestellung in die Verriegelungsstellung. Außerdem kann eine zuverlässige Relativpositionierung des Fixierelementes und des Verriegelungselementes zueinander sichergestellt werden. Ferner ist dadurch das Implantat sicher an der Handhabungsvorrichtung halterbar.

Vorzugsweise sind das Verriegelungselement und/oder ein mit dem Verriegelungselement zum Verriegeln des Implantates zusammenwirkendes Fixierelement der zweiten Implantatkomponente in einer quer zur Verriegelungsrichtung und quer zur Entkopplungsrichtung ausgerichteten Richtung beim Überführen des Verriegelungselementes von der Freigabestellung in die Verriegelungsstellung spielfrei und insbesondere formschlüssig in der Aufnahme angeordnet. Dies ermöglicht ebenfalls eine zuverlässigere Überführung des Verriegelungselementes von der Freigabestellung in die Verriegelungsstellung. Außerdem kann eine zuverlässige Relativpositionierung des Fixierelementes und des Verriegelungselementes zueinander sichergestellt werden. Ferner ist dadurch das Implantat sicher an der Handhabungsvorrichtung halterbar.

Von Vorteil ist es, wenn die Aufnahme erweiterbar ist. Dies vereinfacht es beispielsweise, die Handhabungsvorrichtung am Implantat anzubringen. Wird die Aufnahme erweitert, können so das Verriegelungselement und gegebenenfalls das Fixierelement auf einfachere Weise in die Aufnahme eingeführt werden.

Es kann insbesondere vorgesehen sein, dass die Aufnahme in einer quer zur Verriegelungsrichtung und quer zur Entkopplungsrichtung ausgerichteten Richtung erweiterbar ist.

Bevorzugt umfasst die Handhabungsvorrichtung einen das Verriegelungselement und das Fixierelement an einer ersten Seite derselben übergreifenden ersten Arm sowie einen das Verriegelungselement und das Fixierelement an einer der ersten Seite gegenüberliegenden zweiten Seite derselben untergreifenden zweiten Arm, wobei der erste Arm und der zweite Arm die Aufnahme jeweils zumindest teilweise begrenzen. Der erste und der zweite Arm können das Verriegelungselement und das Fixierelement übergreifen bzw. untergreifen und zwischen sich die Aufnahme definieren. Zwischen dem ersten Arm und dem zweiten Arm sind das Verriegelungselement und das Fixierelement vorzugsweise spielfrei und insbesondere formschlüssig angeordnet. Dies gibt die Möglichkeit, das Implantat mittels der Handhabungsvorrichtung zu halten, etwa um es zu applizieren. Der erste Arm und der zweite Arm können ferner jeweils ein Koppelelement ausbilden zur Kopplung des Fixierelementes jeweils mit mindestens einem zweiten Kopplungsglied, welches jeweils vom ersten Arm und/oder vom zweiten Arm umfasst werden kann. Dies ermöglicht eine Kopplung des Fixierelementes mit dem mindestens einen zweiten Kopplungsglied.

Günstig ist es, wenn der erste Arm und der zweite Arm das Verriegelungselement und das Fixierelement jeweils an einem ersten Ende über- bzw. untergreifen und an einem dem ersten Ende abgewandten jeweiligen zweiten Ende miteinander verbunden sind. Die Handhabungsvorrichtung kann in diesem Fall beispielsweise eine klammerförmige Gestalt aufweisen mit dem ersten Arm und dem zweiten Arm, die zwischen sich die Aufnahme für das Verriegelungselement und das Fixierelement bilden und die miteinander verbunden sind, z.B. mittels eines Steges. Dies gibt die Möglichkeit, den ersten Arm relativ zum zweiten Arm zu spreizen und dadurch die Aufnahme zu erweitern. Dies ermöglicht es, die Handhabungsvorrichtung auf einfachere Weise am Implantat anzubringen.

Vorteilhafterweise ist die Handhabungsvorrichtung am Implantat vormontiert, denn dies vereinfacht dessen Handhabung.

Die Handhabungsvorrichtung kann Bestandteil des Implantats sein und von diesem umfasst sein, insbesondere wenn sie daran vormontiert ist.

Günstig ist es, wenn die Handhabungsvorrichtung eine Verbindungseinrichtung zum Verbinden mit einem ein Griffelement umfassenden Applikator umfasst. Dies ermöglicht es, den Applikator mit der Handhabungsvorrichtung zu verbinden. Der Operateur kann den Applikator am Griffelement erfassen, um die Handhabungsvorrichtung zu betätigen. Ist wie vorstehend beschrieben das Verriegelungselement zusammen mit dem Fixierelement in der Aufnahme der Handhabungsvorrichtung angeordnet und insbesondere darin gehalten, kann der Operateur mittels des Applikators auch das Implantat applizieren. Zudem kann er am Applikator angreifen, um das Verriegelungselement von der Freigabestellung in die Verriegelungsstellung zu überführen.

Bei einer einfachen konstruktiven Ausgestaltung ist die Verbindungseinrichtung als Rasteinrichtung und insbesondere als lösbare Rasteinrichtung ausgebildet.

Eine besonders einfache Konstruktion erhält die Handhabungsvorrichtung, wenn sie einstückig ausgebildet ist.

Von Vorteil ist es, wenn die Handhabungsvorrichtung mindestens ein drittes Angriffsglied umfasst oder ausbildet zur Beaufschlagung des Verriegelungselementes mit einer in Spreizrichtung gerichteten Spreizkraft und zur Anlage an mindestens ein von dem Verriegelungselement umfassten oder ausgebildeten ersten Angriffsglied beim Überführen des Implantates von der Einbringstellung in die Spreizstellung. Das mindestens eine dritte Angriffsglied kann durch Kraftbeaufschlagung der Handhabungsvorrichtung eine Spreizkraft an das Verriegelungselement und insbesondere dessen mindestens eine erste Angriffsglied übertragen. Über das mindestens eine zweite Angriffsglied des Verriegelungselementes kann diese Spreizkraft auf die Implantatkomponente übertragen werden. Dadurch bildet die Handhabungsvorrichtung ein Lagerelement zum Spreizen des Implantates.

Vorzugsweise begrenzt das mindestens eine dritte Angriffsglied zumindest teilweise eine von der Handhabungsvorrichtung umfasste Aufnahme für das Verriegelungselement, denn dies vereinfacht die Konstruktion der Handhabungsvorrichtung.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine perspektivische Darstellung eines erfindungsgemäßen Implantates in einer Einbringstellung;
- Figur 2:: eine perspektivische Darstellung des Implantates aus Figur 1 in einer Spreizstellung, wobei ein Verriegelungselement des Implantates eine Freigabestellung einnimmt;
- Figur 3:: das Implantat aus Figur 2, wobei das Verriegelungselement eine Verriegelungsstellung einnimmt;
- Figur 4:: eine vergrößerte Draufsicht auf ein Fixierelement, das Verriegelungselement und einen Zuganker des Implantates aus Figur 2;
- Figur 5:: eine vergrößerte Draufsicht auf das Fixierelement, das Verriegelungselement und den Zuganker des Implantates aus Figur 3;
- Figur 6:: eine perspektivische Darstellung eines erfindungsgemäßen chirurgischen Systems, umfassend das Implantat aus Figur 1 und eine an diesem angebrachte Handhabungsvorrichtung;
- Figur 7:: eine teilweise Seitenansicht eines endseitigen Bereiches des chirurgischen Systems aus Figur 6;
- Figur 8:: eine Schnittansicht längs der Linie 8-8 in Figur 7;
- Figur 9:: eine perspektivische Teildarstellung des chirurgischen Systems aus Figur 6 beim Abziehen der Handhabungsvorrichtung vom Implantat und
- Figur 10:: eine Seitenansicht des chirurgischen Systems ähnlich Figur 7 beim Abziehen der Handhabungsvorrichtung vom Implantat.

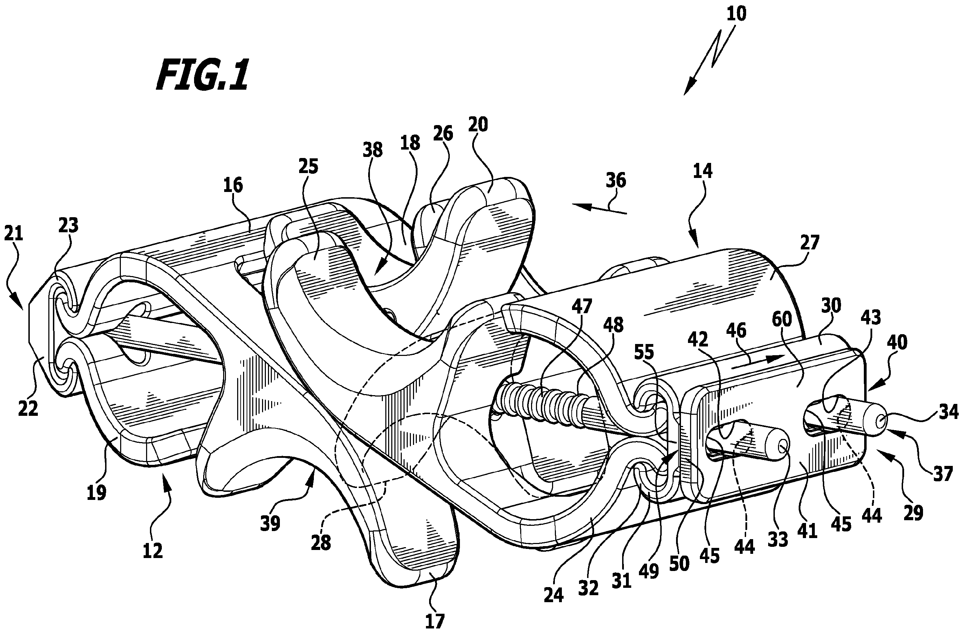

Ein in den Figuren 1 bis 3 perspektivisch dargestelltes Implantat 10 ist ein Zwischenwirbelimplantat zur gegenseitigen Abstützung der Dornfortsätze von in der Zeichnung nicht gezeigten benachbarten Wirbelkörpern. Es gehört zu einer Klasse von in der DE 20 2008 009 344 U1 beschriebenen Implantaten.

Das Implantat umfasst eine erste Implantatkomponente 12 sowie eine zu dieser im Wesentlichen identisch ausgebildete, zweite Implantatkomponente 14. Die erste Implantatkomponente 12 umfasst ein erstes Stützsegment 16, das zwei Stützarme 17 und 18 umfasst, sowie ein zweites Stützsegment 19, das einen zwischen dem ersten Stützarm 17 und dem zweiten Stützarm 18 angeordneten Stützarm 20 umfasst. Die Stützsegmente 16 und 19 sind an einer ersten Endseite 21 des Implantates 10 mittels eines Haltegliedes 22 in Form einer C-förmigen Klammer 23 zusammengehalten.

In entsprechender Weise umfasst die zweite Implantatkomponente 14 ein erstes Stützsegment 24 mit zwei voneinander beabstandeten Stützarmen 25 und 26 sowie ein zweites Stützsegment 27 mit einem zwischen den Stützarmen 25 und 26 angeordneten Stützarm 28. Die Stützsegmente 24 und 27 sind an einer der Endseite 21 gegenüberliegenden Endseite 29 des Implantates 10 mittels eines Haltegliedes 30 in Form einer C-förmigen Klammer 31 zusammengehalten. Das Halteglied 30 wird auch als Fixierelement 32 der Implantatkomponente 14 bezeichnet.

Die Implantatkomponenten 12 und 14 sind relativ zueinander spiegelsymmetrisch bezüglich eines in der Zeichnung nicht dargestellten Symmetriezentrums ausgebildet. Dies hat zur Folge, dass die Stützarme 17 und 25, die Stützarme 18 und 26 sowie die Stützarme 20 und 28 aneinander anliegen können.

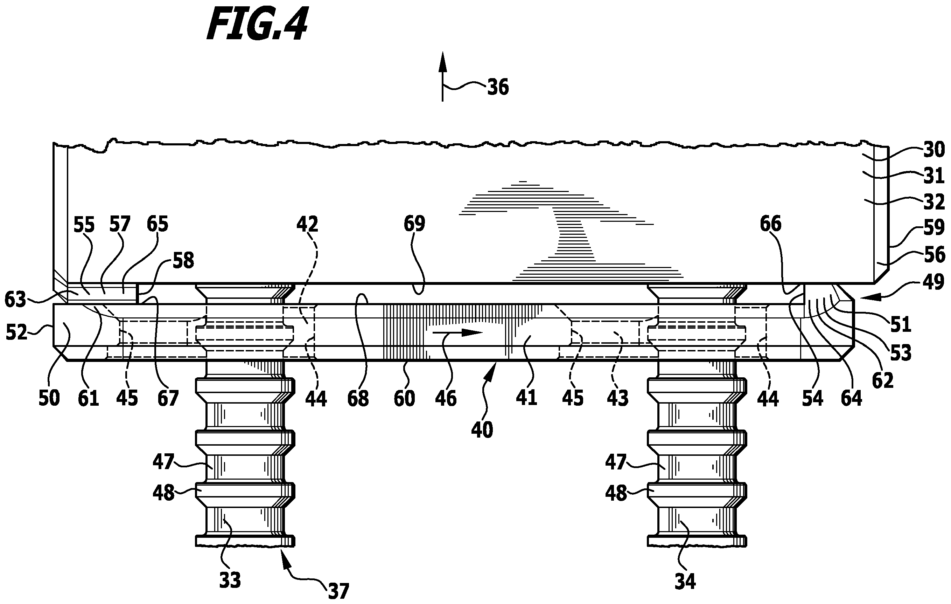

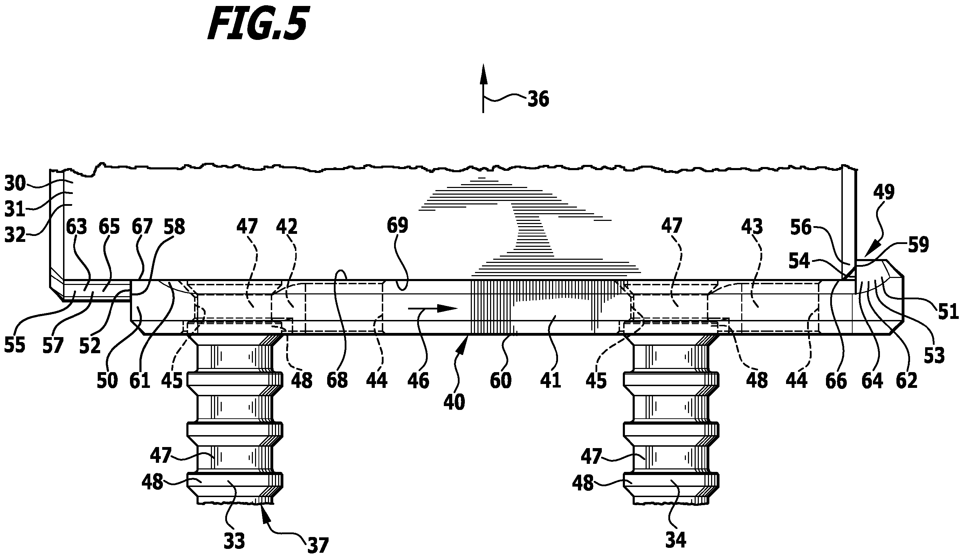

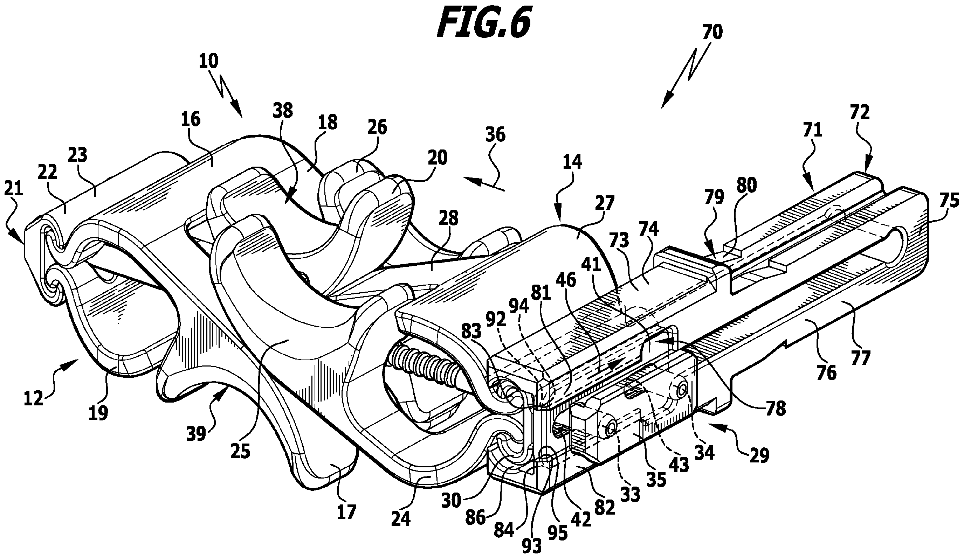

Zwei an der Klammer 23 festgelegte Spannelemente 33 und 34 reichen von der Endseite 21 bis zur Endseite 29 und durchgreifen dabei das Implantat 10 und insbesondere die Klammer 31 an in der Zeichnung nicht dargestellten Öffnungen. Die Spannelemente 33 und 34 sind an der Endseite 29 mittels eines Verbindungsgliedes 35 verbunden, das aus Gründen der Übersichtlichkeit nur in Figur 6 dargestellt ist. Ihre Erstreckungsrichtung definiert eine Spreizrichtung 36 des Implantates 10 (Figuren 4 und 5), und gemeinsam bilden sie einen Zuganker 37 des Implantates 10.

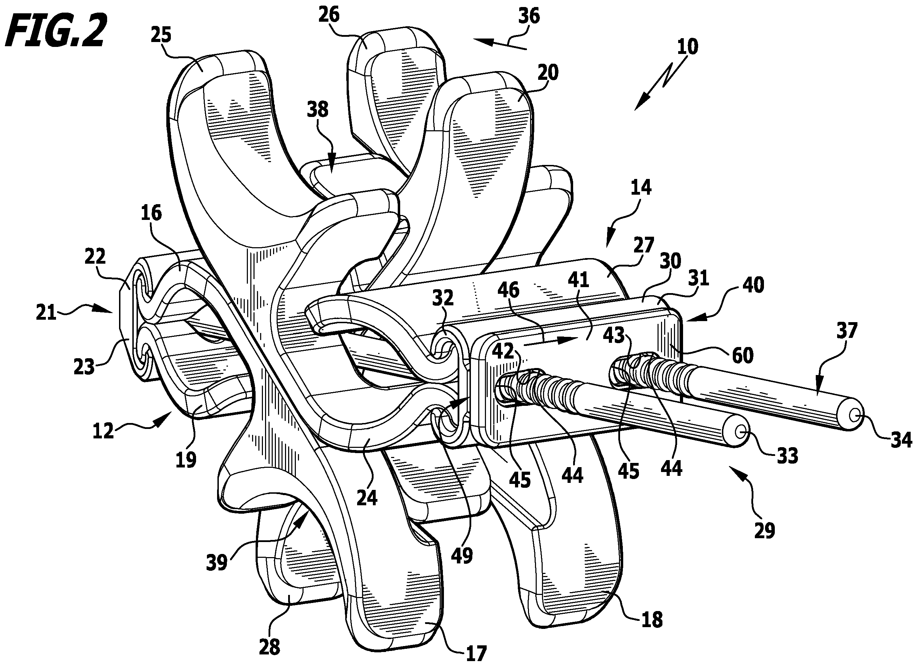

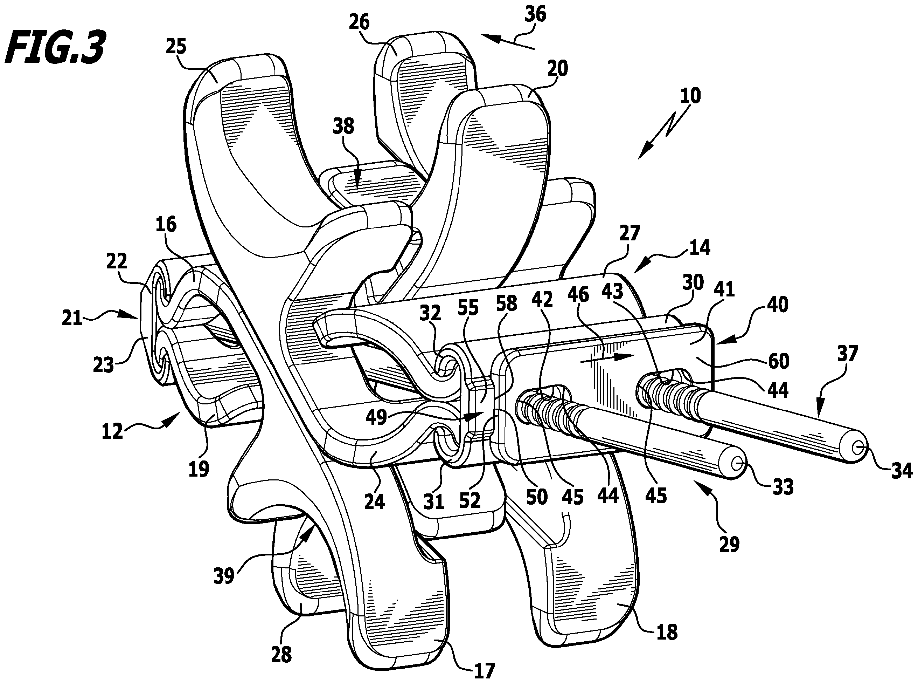

Das Implantat 10 kann ausgehend von einer in Figur 1 dargestellten Einbringstellung durch Kraftbeaufschlagung beispielsweise der Klammer 31 in der Spreizrichtung 36 in eine in den Figuren 2 und 3 dargestellte Spreizstellung überführt werden, indem die zweite Implantatkomponente 14 relativ zur ersten Implantatkomponente 12 entlang des Zugankers 37 verschoben wird. Dabei gleiten die Stützarme 17 und 25, die Stützarme 18 und 26 sowie die Stützarme 20 und 28 jeweils aneinander entlang, so dass sich das Implantat 10 in einer senkrecht zur Spreizrichtung 36 ausgerichteten Richtung spreizt. Eine von den Stützarmen 20, 25 und 26 gebildete obere Stützfläche 38 sowie eine von den Stützarmen 17, 18 und 28 gebildete untere Stützfläche 39 für einen oberen bzw. einen unteren Dornfortsatz weisen in dieser Spreizstellung des Implantates 10 voneinander einen größeren Abstand auf als in der Einbringstellung. Dies ermöglicht es, das Implantat 10 in der Einringstellung durch eine lediglich kleine Körperöffnung zwischen die Wirbelkörper einzubringen und anschließend zur Abstützung der Dornfortsätze von der Einbringstellung in die Spreizstellung zu überführen.

Um das Implantat 10 in der Spreizstellung zu verriegeln, umfasst es eine Verriegelungseinrichtung 40 mit einem plattenförmigen Verriegelungselement 41, welches auf der der Endseite 29 zugewandten Seite der Klammer 31 angeordnet ist und eine senkrecht zur Spreizrichtung 36 orientierte Ebene definiert. Das Verriegelungselement 41 weist zwei Durchbrechungen 42 und 43 auf. Die Durchbrechungen 42 und 43 umfassen jeweils einen ersten Abschnitt 44 und einen zweiten Abschnitt 45 (Figuren 1 bis 3), wobei der erste Abschnitt 44 jeweils so groß ist, dass er von einem der Spannelemente 33 oder 34 durchgriffen werden kann. Der zweite Abschnitt 45 weist einen gegenüber dem ersten Abschnitt 44 verringerten Querschnitt auf.

Nimmt das Implantat 10 die Spreizstellung ein, ist auf diese Weise die Möglichkeit gegeben, das Verriegelungselement 41 in einer quer und insbesondere senkrecht zur Spreizrichtung 36 ausgerichteten Verriegelungsrichtung 46 relativ zum Zuganker 37 so zu verschieben, dass der jeweilige zweite Abschnitt 45 der Durchbrechungen 43 und 44 in Zwischenräume 47 eingreifen kann, die zwischen an den Spannelementen 33 und 34 angeordneten Verriegelungsgliedern 48 gebildet sind.

Die Verriegelungsglieder in Form von Verriegelungsvorsprüngen 48 bilden Anschläge für das Verriegelungselement 41, welches sich auf diese Weise an der ersten Implantatkomponente 12 entgegen der Spreizrichtung 36 abstützen kann. Dadurch kann die zweite Implantatkomponente 14 an der ersten Implantatkomponente 12 verriegelt werden. Dies bedeutet, dass die zweite Implantatkomponente 14 in der auf diese Weise definierten Verriegelungsstellung des Verriegelungselementes 41 gegen eine Bewegung entgegen der Spreizrichtung 36 an der ersten Implantatkomponente 12 verriegelt ist (Figuren 3 und 5).

In die Verriegelungsstellung kann das Verriegelungselement 41 aus einer Freigabestellung überführt werden durch Verschiebung entlang der Verriegelungsrichtung 46, wobei in der Freigabestellung die Implantatkomponenten 12 und 14 entlang der Spreizrichtung 36 und entgegen dieser relativ zueinander beweglich sind (Figuren 1, 2 und 4). Dabei durchgreifen die Spannelemente 33 und 34 die ersten Abschnitte 44 der Durchbrechungen 42 bzw. 43.

Um das Verriegelungselement 41 nach dem Einnehmen der Verriegelungsstellung in derselben gegen eine der Verriegelungsrichtung 46 entgegen gerichteten Bewegung zu sichern und damit eine Entriegelung des Implantates 10 zu verhindern, umfasst das Implantat 10 eine Sicherungseinrichtung 49.

Die Sicherungseinrichtung 49 umfasst ein Paar erster Sicherungsglieder 50 und 51, die einstückig mit dem Verriegelungselement 41 ausgebildet sind. Am Verriegelungselement 41 sind sie an dessen einander entgegengesetzten Enden angeordnet, und zwar das Sicherungsglied 50 im Bereich des Spannelementes 33 und das Sicherungsglied 51 im Bereich des Spannelementes 34. Das Sicherungsglied 50 umfasst einen quer zur Verriegelungsrichtung 46 wirksamen Anschlag 52, und das Sicherungsglied 51 ist als Vorsprung 53 des Verriegelungselementes 41 mit einem quer zur Verriegelungsrichtung 46 wirksamen Anschlag 54 ausgebildet.

In entsprechender Weise umfasst das Implantat 10, und insbesondere die zweite Implantatkomponente 14, speziell die Klammer 31, ein Paar zweiter Sicherungsglieder 55 und 56. Die Sicherungsglieder 55 und 56 sind einstückig mit der Klammer 31 ausgebildet. Sie sind, bezogen auf die Verriegelungsrichtung 46, an einander gegenüberliegenden Enden der Klammer 31 angeordnet, und zwar das Sicherungsglied 55 im Bereich des Spannelementes 33 gegenüber dem Sicherungsglied 50, und das Sicherungsglied 56 im Bereich des Spannelementes 34 gegenüber dem Sicherungsglied 51. Das Sicherungsglied 55 ist als von der Klammer 31 in Richtung des Verriegelungselementes 41 hervorstehender Vorsprung 57 mit einem quer zur Verriegelungsrichtung 46 wirksamen Anschlag 58 ausgebildet, und das Sicherungsglied 56 umfasst einen quer zur Verriegelungsrichtung 46 wirksamen Anschlag 59.

Solange das Verriegelungselement 41 die Freigabestellung einnimmt und das Implantat 10 nicht verriegelt ist, kann es über das Sicherungsglied 51 an der Klammer 31 anliegen und sich entlang der Spreizrichtung 36 daran abstützen, und die Klammer 31 kann über das Sicherungsglied 55 am Verriegelungselement 41 anliegen und sich an diesem entgegen der Spreizrichtung 36 abstützen (Figur 4). Dies kann beispielsweise automatisch erfolgen, wenn das Verriegelungselement 41 zum Spreizen des Implantates 10 mit einer in Spreizrichtung 36 gerichteten Spreizkraft beaufschlagt wird. Hierzu bildet das Verriegelungselement 41 speziell an seiner der Klammer 31 abgewandten Seite ein flächiges Angriffsglied 60 aus. Beim Überführen des Implantates 10 von der Einbringstellung in die Spreizstellung bilden das Sicherungsglied 51 und der dem Sicherungsglied 55 gegenüberliegende Abschnitt 61 des Verriegelungselementes 41 jeweils ein zweites Angriffsglied 62 bzw. 63. Über die Angriffsglieder 62 und 63 kann die am Angriffsglied 60 angreifende Spreizkraft auf die Klammer 31 übertragen werden und damit das Spreizen des Implantates 10, d.h. dessen Überführen von der Einbringstellung in die Spreizstellung, ermöglicht werden.

Wird in der Spreizstellung des Implantates 10 das Verriegelungselement 41 von der Freigabestellung in die Verriegelungsstellung überführt, bilden die Sicherungsglieder 51 und 55 Führungsglieder 64 bzw. 65, die mit durch die Klammer 31 und das Verriegelungselement 41 gebildeten Führungsgliedern 66 bzw. 67 zusammenwirken (Figur 4). Dies sichert eine zuverlässige Überführung des Verriegelungselementes 41 von der Freigabestellung in die Verriegelungsstellung.

Wenn das Verriegelungselement 41 die Verriegelungsstellung einnimmt, sind die Sicherungsglieder 50 und 55 sowie 51 und 56 jeweils entlang der Spreizrichtung 36 relativ zueinander beweglich. Dies erfolgt dadurch, dass das Verriegelungselement 41 relativ zur Klammer 31 genau so weit auf den Zuganker 37 aufgeschoben werden kann, dass die Anschläge 52 und 58 sowie die Anschläge 54 und 59 entlang der Verriegelungsrichtung 46 genau nebeneinander angeordnet werden können.

Für eine kurze Zeitdauer nehmen ein der Klammer 31 zugewandtes Anlageelement 68 des Verriegelungselementes 41 und ein dem Verriegelungselement 41 zugewandtes Anlageelement 69 der Klammer 31 einen Abstand voneinander ein, der durch die Größe der Vorsprünge 53 und 57, d.h. der Sicherungsglieder 51 und 55, definiert wird.

Nach Ablauf der kurzen Zeitdauer können sich die Sicherungsglieder 50 und 55 relativ zueinander sowie 51 und 56 relativ zueinander entlang der Spreizrichtung 36, die mithin auch eine Sicherungsrichtung definiert, bewegen, so dass die Anschläge 52 und 58 bzw. 54 und 59 aneinander zur Anlage gelangen. Dadurch wirken die Sicherungsglieder 50 und 55 sowie 51 und 56 zur Sicherung des Verriegelungselementes 41 in der Verriegelungsstellung gegen eine der Verriegelungsrichtung 46 entgegen gerichtete Bewegung zusammen (Figur 5). Dies definiert eine gesicherte Stellung der Sicherungsglieder 50, 51, 55 und 56, und eine Stellung der Sicherungseinrichtung 49, in der die Sicherungsglieder 50 und 55 sowie 51 und 56 nicht zusammenwirken, definiert eine entsicherte Stellung der Sicherungsglieder 50, 51, 55 und 56. In der gesicherten Stellung hintergreifen sich das Verriegelungselement 41 und die Klammer 31 gegenseitig, so dass das Implantat 10 verriegelt und zudem das Verriegelungselement 41 in der Verriegelungsstellung gesichert ist.

Die Relativbewegung der Sicherungsglieder 50 und 55 sowie 51 und 56 zueinander kann mit dem Einnehmen der Verriegelungsstellung durch das Verriegelungselement 41 automatisch erfolgen. Dies ist dadurch bedingt, dass das Verriegelungselement 41 die Verriegelungsstellung erst dann einnehmen kann, wenn das Implantat 10 sich in der Spreizstellung befindet, weil sich die Verriegelungsvorsprünge 48 nicht über die gesamte Länge des Zugankers 37 erstrecken, sondern nur an einem mittleren Bereich desselben zwischen den Endseiten 21 und 29 angeordnet sind. In der Spreizstellung sind die Implantatkomponenten 12 und 14 nun entlang der Spreizrichtung 36 gegeneinander vorgespannt. Dies ist dadurch begründet, dass die Stützsegmente 16, 19, 24 und 27 aus einem elastischen Material gebildet sind und sich gegeneinander abstützen. Dadurch entsteht eine Vorspannkraft, die einerseits an der ersten Implantatkomponente 12, dadurch am Zuganker 37 und dadurch am Verriegelungselement 41 angreift sowie andererseits an der zweiten Implantatkomponente 14 und damit an der Klammer 31. Unmittelbar nach dem Einnehmen der Verriegelungsstellung durch das Verriegelungselement 41 bewegen sich dadurch die Klammer 31 und das Verriegelungselement 41 aufeinander zu und damit die Sicherungsglieder 50 und 55 sowie 51 und 56 aufeinander zu, um die automatische Sicherung des Verriegelungselementes 41 zu gewährleisten.

Selbst bei einem nicht elastisch ausgebildeten Implantat könnte eine über die Stützflächen 38 und 39 auf die Implantatkomponenten 12 und 14 eingeleitete Stützkraft zu einer entlang der Spreizrichtung 36 wirkenden Stützkraft führen, mit der sich ein automatisches Sichern des Verriegelungselementes 41 in der Verriegelungsstellung gewährleisten ließe.

Eine bevorzugte Ausführungsform eines erfindungsgemäßen chirurgischen Systems ist in Figur 6 perspektivisch dargestellt und dort mit dem Bezugszeichen 70 belegt. Das System 70 umfasst das vorstehend beschriebene erfindungsgemäße Implantat 10 sowie eine Handhabungsvorrichtung 71, mit der u.a. das Verriegelungselement 41 von der Freigabestellung in die Verriegelungsstellung überführbar ist. Die Handhabungsvorrichtung 71 wird nachfolgend als Adapter 72 bezeichnet.

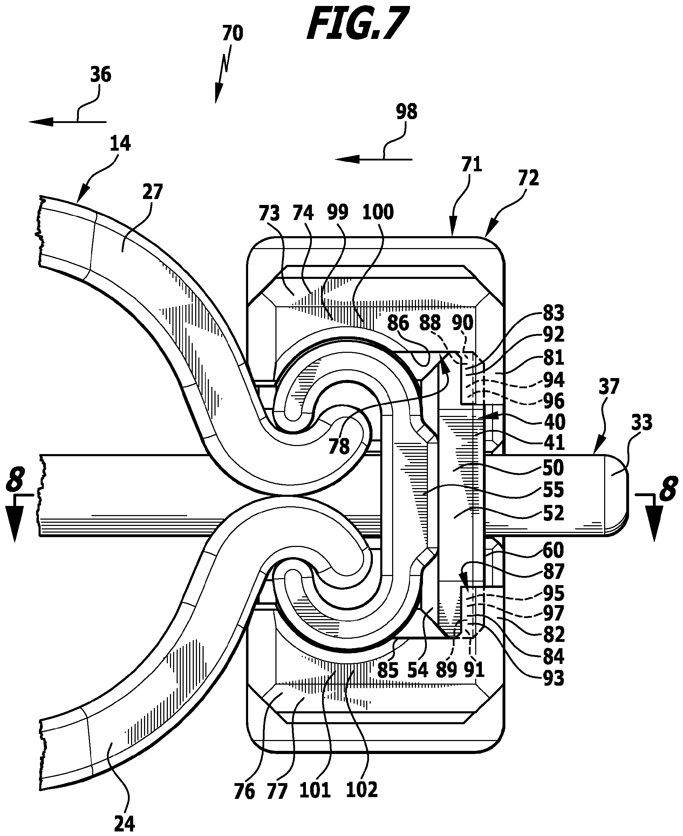

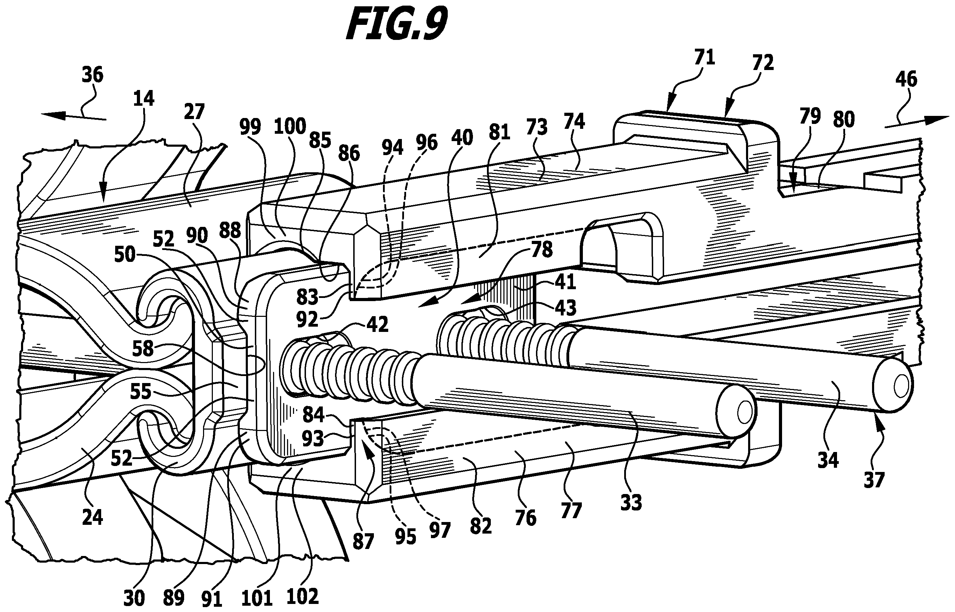

Der Adapter 72 umfasst einen ein erstes Halteelement 73 für das Implantat 10 bildenden ersten Arm 74, der die Klammer 31 und das Verriegelungselement 41 übergreifen kann. Seine der Klammer 31 und dem Verriegelungselement 41 zugewandte Unterseite ist der Kontur der Klammer 31 und der Kontur des Verriegelungselementes 41 entsprechend ausgebildet (Figuren 7 und 9), so dass das Verriegelungselement 41 und die Klammer 31 flächig am ersten Arm 74 anliegen können. Er steht vom Implantat 10 entlang der Verriegelungsrichtung 46 ab und ist an seinem dem Implantat 10 abgewandten Ende über eine stegförmigen Verbindungsabschnitt 75 mit einem ein weiteres Halteelement 76 bildenden zweiten Arm 77 des Adapters 72 verbunden.

Der zweite Arm 77 ist bezüglich einer durch den Zuganker 37 definierten Ebene relativ zum ersten Arm 74 spiegelsymmetrisch ausgebildet, so dass der zweite Arm 77 die Klammer 31 und das Verriegelungselement 41 untergreifen und insbesondere flächig an diesem anliegen kann.

Auf vorstehend beschriebene Weise bildet der Adapter 72 gewissermaßen eine Klammer mit den beiden Armen 74 und 77, die zwischen sich eine Aufnahme 78 für die Klammer 31 und das Verriegelungselement 41 definieren. In der Aufnahme 78 sind die Klammer 31 und das Verriegelungselement 41 senkrecht zur Spreizrichtung 36 und senkrecht zur Verriegelungsrichtung 46 spielfrei und insbesondere formschlüssig angeordnet. Dies gibt die Möglichkeit, das Implantat 10 mittels des Adapters 72 zu halten, so dass es auch mit diesem in den Zwischenwirbelraum eingebracht werden kann.

Der Adapter 72 ist einstückig ausgebildet. Er kann mit dem Implantat 10 im Auslieferungszustand verbunden sein, d.h. auf diesem vormontiert sein. Es ist auch möglich, dass er nachträglich auf das Implantat 10 aufgesetzt oder an diesem angebracht wird. Hierzu können die Arme 74 und 77 gegeneinander senkrecht zur Spreizrichtung 36 und senkrecht zur Verriegelungsrichtung 46 gespreizt werden, weil sie miteinander nur mittels des verhältnismäßig dünnen stegförmigen Verbindungsabschnittes 75 verbunden sind. Dies gibt die Möglichkeit, die Aufnahme 78 so zu erweitern, dass der Adapter 72 auf das Implantat 10 aufgesetzt oder daran angebracht werden kann.

Etwa mittig zwischen dem Verbindungsabschnitt 75 und der Aufnahme 78 umfasst der Adapter 72 eine Verbindungseinrichtung 79 in Form einer Rasteinrichtung 80. Auf die Rasteinrichtung 80 kann eine korrespondierend zu dieser ausgebildete Rasteinrichtung eines Applikators aufgerastet werden, um den Adapter 72 mit dem Applikator zu verbinden. Ein zu verbindender Applikator umfasst günstigerweise ein Griffelement, um einem Operateur ein einfaches Hantieren mit dem Adapter 72 und dem daran gehaltenen Implantat 10 zu ermöglichen.

Der Adapter 72 bildet ein erstes Lagerelement zum Spreizen des Implantates 10 aus. Hierfür umfasst er zwei Angriffsglieder 81 und 82, die am ersten Arm 74 bzw. am zweiten Arm 77 gebildet sind. Sie begrenzen die Aufnahme 78 teilweise in Richtung der Endseite 29 des Implantates 10, wenn der Adapter 72 am Implantat 10 angebracht ist. Die Angriffsglieder 81 und 82 können das vom Verriegelungselement 41 gebildete Angriffsglied 60 mit einer in Spreizrichtung 36 gerichteten Spreizkraft beaufschlagen. Nimmt das Verriegelungselement 41 die Freigabestellung ein, kann diese Spreizkraft über die Angriffsglieder 62 und 63 auf die Klammer 31 und damit die zweite Implantatkomponente 14 übertragen werden. Durch Bewegen des Adapters 72 entlang der Spreizrichtung 36 kann dieser damit zum Überführen des Implantates 10 von der Einbringstellung in die Spreizstellung als Lagerelement genutzt werden.

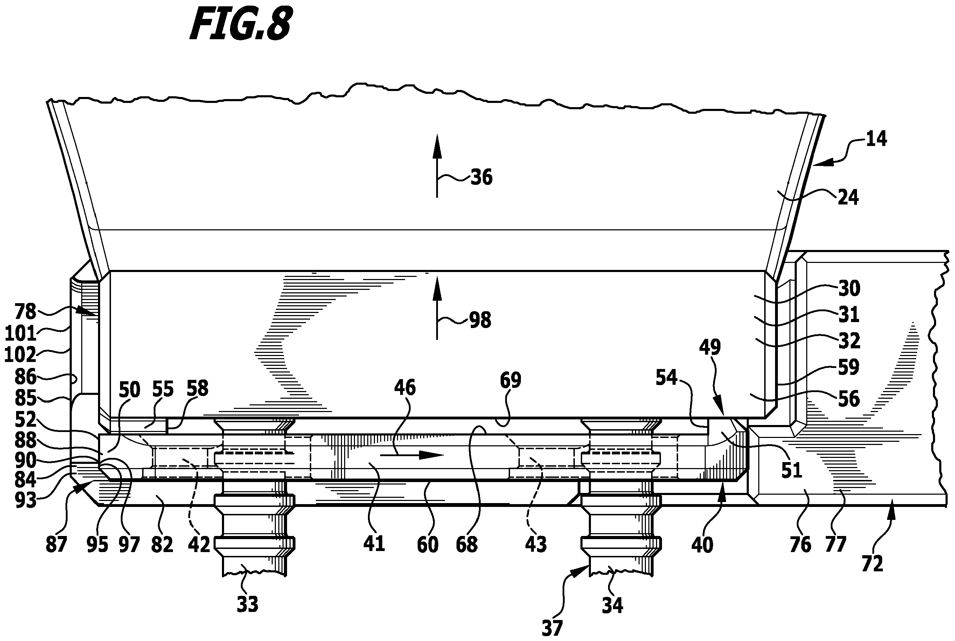

Entlang der Spreizrichtung 36 ist die Aufnahme 78 des Adapters 72 so bemessen, dass jedenfalls dann, wenn das Verriegelungselement 41 die Freigabestellung einnimmt, das Verriegelungselement 41 und die Klammer 31 entlang der Spreizrichtung 36 spielfrei und insbesondere formschlüssig in der Aufnahme 78 angeordnet sind (Figuren 7 und 8). Dies ermöglicht ein zuverlässiges Halten des Implantates 10 am Applikator 72 und ein zuverlässiges Überführen des Implantates 10 von der Einbringstellung in die Spreizstellung.

Entlang der Verriegelungsrichtung 46 ist das Verriegelungselement 41 in der Freigabestellung spielfrei und insbesondere formschlüssig in der Aufnahme 78 gehalten. Der Adapter 72 umfasst hierfür ein erstes Anschlagelement 83 am ersten Arm 74 sowie ein zweites Anschlagelement 84 am zweiten Arm 77. Die Anschlagelemente 83 und 84 sind jeweils an einem Rand 85 einer Öffnung 86 der Aufnahme 78 angeordnet. Die Öffnung 86 ist auf der dem Verbindungsabschnitt 75 gegenüberliegenden Ende des Adapters 72 gebildet. Somit begrenzen die Anschlagelemente 83 und 84 die Öffnung 86 jeweils abschnittsweise.

Die Öffnung 86 ist entlang der Spreizrichtung 36 so bemessen, dass dann, wenn sich das Verriegelungselement 41 in der Freigabestellung befindet, wobei die Anlageelemente 68 und 69 einen Abstand zueinander einnehmen, die Klammer 31 und das Verriegelungselement 41 nicht aus der Aufnahme 78 entfernt werden können. Demgegenüber ist die Öffnung 86 gerade so groß, dass dann, wenn die Anlageelemente 68 und 69 aneinander anliegen, die Klammer 31 und das Verriegelungselement 41 durch die Öffnung 86 aus der Aufnahme 78 entfernt werden können. Dies bedeutet, dass dann, wenn die Sicherungsglieder 50, 51, 55 und 56 die gesicherte Stellung einnehmen und das Verriegelungselement 41 gegen eine der Verriegelungsrichtung 46 entgegen gerichtete Bewegung gesichert ist, ein Entfernen der Klammer 31 und des Verriegelungselementes 41 aus der Aufnahme 78 möglich ist, aber nicht vorher. Konsequenterweise können die Klammer 31 und das Verriegelungselement 41 somit auch dann, wenn das Verriegelungselement 41 zwar die Verriegelungsstellung einnimmt, die Sicherungsglieder 50, 51, 55 und 56 aber noch die entsicherte Stellung einnehmen, nicht aus der Aufnahme 78 entfernt werden, denn auch in diesem Fall weisen die Anlageelemente 68 und 69 noch einen Abstand voneinander auf. In letzterem Fall sind das Verriegelungselement 41 auch in der Verriegelungsstellung und die Klammer 31 entlang der Spreizrichtung 36 noch spielfrei und insbesondere formschlüssig in der Aufnahme 78 angeordnet.

Über die bereits beschriebenen Funktionen hinaus dient der Adapter 72 zum Überführen des Verriegelungselementes 41 von der Freigabestellung in die Verriegelungsstellung. Hierfür umfasst das chirurgische System 70 eine Kopplungseinrichtung 87. Die Kopplungseinrichtung 87 umfasst zwei erste Kopplungsglieder 88 und 89, die jeweils vom Verriegelungselement 41 umfasst werden und einstückig mit diesem verbunden sind. Sie sind an dem das Sicherungsglied 50 aufweisenden Ende des Verriegelungselementes 41 angeordnet und umfassen quer zur Verriegelungsrichtung 46 wirksame Anschläge 90 bzw. 91 (Figur 9).

Weiter umfasst die Kopplungseinrichtung 87 zwei zweite Kopplungsglieder 92 und 93, welche durch die Anschlagelemente 83 bzw. 84 ausgebildet sind. Die Kopplungsglieder 92 und 93 umfassen quer zur Verriegelungsrichtung 46 wirksame Anschläge 94 bzw. 95, und sie bilden Mitnehmer 96 bzw. 97 für das Verriegelungselement 41 (Figuren 7 und 8).

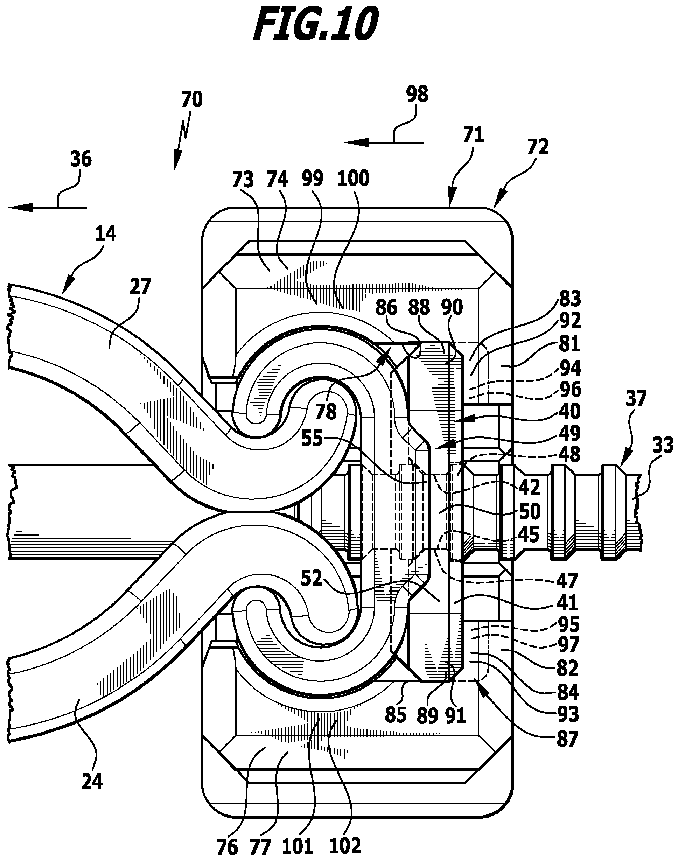

In der Freigabestellung des Verriegelungselementes 41 und in dessen Verriegelungsstellung, solange die Sicherungsglieder 50, 51, 55 und 56 aber noch die entsicherte Stellung einnehmen, können die Kopplungsglieder 88 und 92 sowie 89 und 93 aneinander anliegen, wobei die Anschläge 90 und 94 bzw. 91 und 95 zusammenwirken. Dies definiert eine Kopplungsstellung der Kopplungsglieder 88, 89, 92 und 93. Wird der Adapter 72 mit einer in der Verriegelungsrichtung 46 wirkenden Kraft beaufschlagt, wird das Verriegelungselement 41 unter der Wirkung der Kopplungseinrichtung 87 ebenfalls in der Verriegelungsrichtung 46 bewegt. Dies erfolgt so lange, bis das Verriegelungselement 41 die Verriegelungsstellung einnimmt. Unmittelbar danach werden, wie vorstehend erwähnt, die Sicherungsglieder 50, 51, 55 und 56 von der entsicherten Stellung in die gesicherte Stellung überführt, wobei die Klammer 31 und das Verriegelungselement 41 mittels der Anlageelemente 69 bzw. 68 unter der Wirkung der Vorspannung der Implantatkomponenten 12 und 14 relativ zueinander zur Anlage gelangen (Figur 10).

Wenn das Verriegelungselement 41 die Verriegelungsstellung einnimmt, sind die Kopplungsglieder 88 und 92 sowie 89 und 93 relativ zueinander in einer parallel zur Spreizrichtung 36 ausgerichteten Entkopplungsrichtung 98 beweglich. Dies folgt zum einen daraus, dass die Kopplungsglieder 88 und 89 vom Verriegelungselement 41 umfasst werden, das in der Verriegelungsstellung an der ersten Implantatkomponente 12 gehalten angreift. Zum anderen folgt dies daraus, dass die Arme 74 und 77 die Klammer 31 formschlüssig über- bzw. untergreifen und zugleich das Kopplungsglied 92 bzw. das Kopplungsglied 93 umfassen. Der die Aufnahme 78 obenseitig begrenzende Abschnitt 99 des Armes 74 bildet somit ein Koppelelement 100, und der die Aufnahme 78 untenseitig begrenzende Abschnitt 101 des Armes 77 bildet in entsprechender Weise ein Koppelelement 102. Über die Koppelelemente 100 und 102 sind die Kopplungsglieder 92 bzw. 93 somit mit der Klammer 31 und damit mit der zweiten Implantatkomponente 14 gekoppelt.

Eine Bewegung der Klammer 31 und des Verriegelungselementes 41 aufeinander zu infolge der Vorspannung der Implantatkomponenten 12 und 14 relativ zueinander führt damit automatisch zu einer Relativbewegung der Kopplungsglieder 88 und 92 sowie 89 und 93 zueinander. Dies erfolgt genau dann, wenn die Sicherungsglieder 50, 51, 55 und 56 von der entsicherten Stellung in die gesicherte Stellung überführt werden. In diesem Moment werden die Kopplungsglieder 88 und 92 sowie 89 und 93 relativ zueinander automatisch von der Kopplungsstellung in eine Entkopplungsstellung überführt, in der die Anschläge 90 und 94 bzw. 91 und 95 relativ zueinander einen Abstand aufweisen und infolgedessen nicht mehr zusammenwirken.

Wie im Fall der Sicherungsglieder 50, 51, 55 und 56 beruht diese automatische Entkopplung der Kopplungsglieder 88, 89, 92 und 93 voneinander auf deren Vorspannung relativ zueinander, wenn das Verriegelungselement 41 die Verriegelungsstellung einnimmt. Diese Vorspannung ist auf die Vorspannung der Implantatkomponenten 12 und 14 relativ zueinander in der Spreizstellung des Implantates 10 zurückzuführen.

Stehen die Implantatkomponenten 12 und 14 relativ zueinander in der Spreizstellung des Implantates 10 nicht unter Vorspannung, so kann dennoch eine auf die Stützflächen 38 und 39 einwirkende Stützkraft der Dornfortsätze entlang der Spreizrichtung 36 wirksam werden. Dies führt ebenfalls neben der bereits angesprochenen automatischen Sicherung des Verriegelungselementes 41 zur automatischen Entkopplung des Adapters 72 vom Verriegelungselement 41.

Nimmt das Verriegelungselement 41 die Verriegelungsstellung ein, die Sicherungsglieder 50, 51, 55 und 56 die gesicherte Stellung und die Kopplungsglieder 88, 89, 92 und 93 die Entkopplungsstellung, liegen die Anlageelemente 68 und 69 aneinander an. Die Klammer 31 und das Verriegelungselement 41 können somit durch die Öffnung 86 aus der Aufnahme 78 entfernt werden. Dies erfolgt durch weitere Kraftbeaufschlagung des Adapters 72 in der Verriegelungsrichtung 46. Der Adapter 72 kann dadurch nach dem automatischen Verriegeln des Implantates 10 und dem automatischen Entkoppeln des Verriegelungselementes 41 vom Implantat 10 abgezogen werden (Figur 9).

## Patentansprüche

1. Implantat (10) zur gegenseitigen Abstützung der Dornfortsätze benachbarter Wirbelkörper, mit einer ersten Implantatkomponente (12) und einer zweiten Implantatkomponente (14), wobei das Implantat (10) von einer Einbringstellung, in der das Implantat (10) zwischen einen oberen Dornfortsatz eines oberen Wirbelkörpers und einen unteren Dornfortsatz eines unteren Wirbelkörpers einbringbar ist, durch Bewegen der zweiten Implantatkomponente (14) relativ zur ersten Implantatkomponente (12) entlang einer Spreizrichtung (36) in eine Spreizstellung überführbar ist, in der eine vom Implantat (10) gebildete obere Stützfläche (38) für den oberen Dornfortsatz und eine vom Implantat (10) gebildete untere Stützfläche (39) für den unteren Dornfortsatz einen größeren Abstand relativ zueinander einnehmen als in der Einbringstellung, und mit einer ein Verriegelungselement (41) umfassenden Verriegelungseinrichtung (40), wobei das Verriegelungselement (41) von einer Freigabestellung, in der die zweite Implantatkomponente (14) entgegen der Spreizrichtung (36) relativ zur ersten Implantatkomponente (12) beweglich ist, relativ zu mindestens einer Implantatkomponente (12, 14) entlang einer quer zur Spreizrichtung (36) ausgerichteten Verriegelungsrichtung (46) in eine Verriegelungsstellung überführbar ist, in der die zweite Implantatkomponente (14) gegen eine der Spreizrichtung (36) entgegen gerichtete Bewegung an der ersten Implantatkomponente (12) verriegelt ist, sowie mit einer Sicherungseinrichtung (49) zur Sicherung des Verriegelungselementes (41) gegen eine der Verriegelungsrichtung (46) entgegen gerichtete Bewegung, **dadurch gekennzeichnet, dass** die Sicherungseinrichtung (49) mindestens ein erstes Sicherungsglied (50, 51) aufweist sowie mindestens ein zweites Sicherungsglied (55, 56), die nach dem Einnehmen der Verriegelungsstellung durch das Verriegelungselement (41) relativ zueinander in einer quer zur Verriegelungsrichtung (46) ausgerichteten Sicherungsrichtung von einer entsicherten Stellung, in der das mindestens eine erste Sicherungsglied (50, 51) und das mindestens eine zweite Sicherungsglied (55, 56) nicht zusammenwirken, in eine gesicherte Stellung überführbar sind, in der das mindestens eine erste Sicherungsglied (50, 51) und das mindestens eine zweite Sicherungsglied (55, 56) zur Sicherung des Verriegelungselementes (41) zusammenwirken, dass das Verriegelungselement (41) das mindestens eine erste Sicherungsglied (50, 51) umfasst und eine Implantatkomponente (12, 14) das mindestens eine zweite Sicherungsglied (55, 56) umfasst, und dass das mindestens eine erste Sicherungsglied (50, 51) und das mindestens eine zweite Sicherungsglied (55, 56) in der entsicherten Stellung relativ zueinander entlang der Sicherungsrichtung vorgespannt sind.

2. Implantat nach Anspruch 1, **dadurch gekennzeichnet, dass** mindestens ein Sicherungsglied (50, 51, 55, 56) einen entgegen der Verriegelungsrichtung (46) wirksamen Anschlag (52, 54, 58, 59) umfasst oder ausbildet.

3. Implantat nach Anspruch 1 oder 2, **dadurch gekennzeichnet, dass** das mindestens eine erste Sicherungsglied (50, 51) und das mindestens eine zweite Sicherungsglied (55, 56) in der gesicherten Stellung aneinander anliegen.

4. Implantat nach einem der voranstehenden Ansprüche, **dadurch gekennzeichnet, dass** die zweite Implantatkomponente (14) das mindestens eine zweite Sicherungsglied (55, 56) umfasst.

5. Implantat nach Anspruch 4, **dadurch gekennzeichnet, dass** die zweite Implantatkomponente (14) ein mit dem Verriegelungselement (41) zum Verriegeln des Implantates (10) zusammenwirkendes Fixierelement (32) aufweist, das das mindestens eine zweite Sicherungsglied (55, 56) umfasst.

6. Implantat nach einem der voranstehenden Ansprüche, **dadurch gekennzeichnet, dass** mindestens ein Sicherungsglied (50, 51, 55, 56) und/oder das Verriegelungselement (41) und/oder ein mit dem Verriegelungselement (41) zum Verriegeln des Implantates (10) zusammenwirkendes Fixierelement (32) der zweiten Implantatkomponente (14) starr ausgebildet sind.

7. Implantat nach einem der voranstehenden Ansprüche, **dadurch gekennzeichnet, dass** das Verriegelungselement (41) und die das mindestens eine zweite Sicherungsglied (55, 56) umfassende Implantatkomponente (14) jeweils zwei in der gesicherten Stellung paarweise zusammenwirkende Sicherungsglieder (50, 51, 55, 56) umfassen.

8. Implantat nach einem der voranstehenden Ansprüche, **dadurch gekennzeichnet, dass** das Verriegelungselement (41) und die das mindestens eine zweite Sicherungsglied (55, 56) umfassende Implantatkomponente (14) einander in der gesicherten Stellung hintergreifen.

9. Implantat nach einem der voranstehenden Ansprüche, **dadurch gekennzeichnet, dass** das Verriegelungselement (41) relativ zur ersten Implantatkomponente (12) und/oder relativ zur zweiten Implantatkomponente (14) zur Überführung von der Freigabestellung in die Verriegelungsstellung verschieblich ausgebildet ist.

10. Implantat nach einem der voranstehenden Ansprüche, **dadurch gekennzeichnet, dass** die Sicherungsrichtung senkrecht zur Verriegelungsrichtung (46) ausgerichtet ist.

11. Implantat nach einem der voranstehenden Ansprüche, **dadurch gekennzeichnet, dass** die zweite Implantatkomponente (14) relativ zur ersten Implantatkomponente (12) entgegen der Spreizrichtung (36) in der Verriegelungsstellung des Verriegelungselementes (41) vorgespannt ist.

12. Implantat nach einem der voranstehenden Ansprüche, **dadurch gekennzeichnet, dass** die Sicherungsrichtung eine zur Spreizrichtung (36) parallele Komponente aufweist.

13. Chirurgisches System, umfassend mindestens ein Implantat (10) nach einem der voranstehenden Ansprüche sowie eine Handhabungsvorrichtung (71) zum Überführen des Verriegelungselementes (41) von der Freigabestellung in die Verriegelungsstellung.

14. Chirurgisches System nach Anspruch 13, **dadurch gekennzeichnet, dass** die Handhabungsvorrichtung (71) am Implantat (10) vormontiert ist.

15. Chirurgisches System nach Anspruch 13 oder 14, **dadurch gekennzeichnet, dass** die Handhabungsvorrichtung (71) eine Verbindungseinrichtung (79) zum Verbinden mit einem ein Griffelement umfassenden Applikator umfasst.

## Claims

1. An implant (10) for mutually supporting the spinous processes of adjacent vertebral bodies comprising a first implant component (12) and a second implant component (14), wherein the implant (10) is transferable by moving the second implant component (14) relative to the first implant component (12) along a spreading direction (36) from an introduction position, in which the implant (10) is introducible between an upper spinous process of an upper vertebral body and a lower spinous process of a lower vertebral body, into a spread position, in which an upper supporting surface (38) for the upper spinous process that is formed by the implant (10) and a lower supporting surface (39) for the lower spinous process that is formed by the implant (10) have a greater spacing relative to each other than in the introduction position, and comprising a locking device (40) incorporating a locking element (41) wherein the locking element (41) is transferable relative to at least one implant component (12, 14) along a locking direction (46) oriented transversely to the spreading direction (36) from a release position, in which the second implant component (14) is movable relative to the first implant component (12) in a direction opposed to the spreading direction (36), into a locking position in which the second implant component (14) is locked to the first implant component (12) against a movement that is directed counter to the spreading direction (36), and also comprising a securing device (49) for securing the locking element (41) against a movement that is directed counter to the locking direction (46), **characterized in that** the securing device (49) comprises at least one first securing member (50, 51) and also at least one second securing member (55, 56) which, following adoption of the locking position by the locking element (41), are transferable relative to one another in a securing direction that is directed transversely relative to the locking direction (46) from an unsecured position, in which the at least one first securing member (50, 51) and the at least one second securing member (55, 56) do not co-operate, into a secured position in which the at least one first securing member (50, 51) and the at least one second securing member (55, 56) co-operate for securing the locking element (41), **in that** the locking element (41) comprises the at least one first securing member (50, 51) and an implant component (12, 14) comprises the at least one second securing member (55, 56), and **in that** the at least one first securing member (50, 51) and the at least one second securing member (55, 56) are biased relative to each other along the securing direction in the unsecured position.

2. An implant in accordance with Claim 1, **characterized in that** at least one securing member (50, 51, 55, 56) comprises or forms a stop (52, 54, 58, 59) which acts in a direction opposed to the locking direction (46).

3. An implant in accordance with Claim 1 or 2, **characterized in that** the at least one first securing member (50, 51) and the at least one second securing member (55, 56) abut one another in the secured position.

4. An implant in accordance with any of the preceding Claims, **characterized in that** the second implant component (14) comprises the at least one second securing member (55, 56).

5. An implant in accordance with Claim 4, **characterized in that** the second implant component (14) has a fixing element (32) which co-operates with the locking element (41) for locking the implant (10) and which comprises the at least one second securing member (55, 56).

6. An implant in accordance with any of the preceding Claims, **characterized in that** at least one securing member (50, 51, 55, 56) and/or the locking element (41) and/or a fixing element (32) of the second implant component (14) co-operating with the locking element (41) for locking the implant (10) are rigid.

7. An implant in accordance with any of the preceding Claims, **characterized in that** the locking element (41) and the implant component (14) comprising the at least one second securing member (55, 56) each comprise two securing members (50, 51, 55, 56) which co-operate in pairs in the secured position.

8. An implant in accordance with any of the preceding Claims, **characterized in that** the locking element (41) and the implant component (14) comprising the at least one second securing member (55, 56) engage behind one another in the secured position.

9. An implant in accordance with any of the preceding Claims, **characterized in that** the locking element (41) is formed such as to be displaceable relative to the first implant component (12) and/or relative to the second implant component (14) for transferring from the release position into the locking position.

10. An implant in accordance with any of the preceding Claims, **characterized in that** the securing direction is oriented perpendicularly to the locking direction (46).

11. An implant in accordance with any of the preceding Claims, **characterized in that** the second implant component (14) is biased relative to the first implant component (12) in a direction opposed to the spreading direction (36) in the locking position of the locking element (41).

12. An implant in accordance with any of the preceding Claims, **characterized in that** the securing direction has a component parallel to the spreading direction (36).

13. A surgical system comprising at least one implant (10) in accordance with any of the preceding Claims and also a handling device (71) for transferring the locking element (41) from the release position into the locking position.

14. A surgical system in accordance with Claim 13, **characterized in that** the handling device (71) is pre-mounted on the implant (10).

15. A surgical system in accordance with Claim 13 or 14, **characterized in that** the handling device (71) comprises a connecting device (79) for connection to an applicator comprising a gripping element.

## Revendications

1. Implant (10) pour le soutien réciproque des apophyses épineuses de corps vertébraux voisins, avec un premier composant d'implant (12) et un second composant d'implant (14), où l'implant (10) peut être amené à passer d'une position d'introduction, dans laquelle l'implant (10) peut être introduit entre une apophyse épineuse supérieure d'un corps vertébral supérieur et une apophyse épineuse inférieure d'un corps vertébral inférieur, par déplacement du second composant d'implant (14) par rapport au premier composant d'implant (12) le long d'une direction d'expansion (36), dans une position d'expansion dans laquelle une surface de soutien supérieure (38) pour l'apophyse épineuse supérieure, formée par l'implant (10), et une surface de soutien inférieure (39) pour l'apophyse épineuse inférieure, formée par l'implant (10), occupent une plus grande distance l'une par rapport à l'autre que dans la position d'introduction, et avec un mécanisme de verrouillage (40) comprenant un élément de verrouillage (41), où l'élément de verrouillage (41) peut être amené à passer d'une position de libération dans laquelle le second composant d'implant (14) peut être déplacé à l'encontre de la direction d'expansion (36) par rapport au premier composant d'implant (12), par rapport à au moins un composant d'implant (12, 14) le long d'une direction de verrouillage (46) orientée transversalement par rapport à la direction d'expansion (36) dans une position de verrouillage dans laquelle le second composant d'implant (14) est verrouillé au niveau du premier composant d'implant (12) contre un mouvement dirigé à l'encontre de la direction d'expansion (36), ainsi qu'avec un mécanisme de blocage (49) pour le blocage de l'élément de verrouillage (41) contre un mouvement dirigé à l'encontre de la direction de verrouillage (46), **caractérisé en ce que** le mécanisme de blocage (49) présente au moins un premier membre de blocage (50, 51) ainsi qu'au moins un second membre de blocage (55, 56) qui peuvent être amenés à passer après l'occupation de la position de verrouillage par l'élément de verrouillage (41) l'un par rapport à l'autre dans une direction de blocage orientée transversalement par rapport à la direction de verrouillage (46) d'une position débloquée dans laquelle le au moins un premier membre de blocage (50, 51) et le au moins un second membre de blocage (55, 56) ne coopèrent pas, dans une position bloquée dans laquelle le au moins un premier membre de blocage (50, 51) et le au moins un second membre de blocage (55, 56) coopèrent pour le blocage de l'élément de verrouillage (41), **en ce que** l'élément de verrouillage (41) comprend le au moins un premier membre de blocage (50, 51) et un composant d'implant (12, 14) comprend le au moins un second membre de blocage (55, 56), et **en ce que** le au moins un premier membre de blocage (50, 51) et le au moins un second membre de blocage (55, 56) sont précontraints l'un par rapport à l'autre le long de la direction de blocage dans la position débloquée.

2. Implant selon la revendication 1, **caractérisé en ce qu'**au moins un membre de blocage (50, 51, 55, 56) comprend ou forme une butée (52, 54, 58, 59) active à rencontre de la direction de verrouillage (46).

3. Implant selon la revendication 1 ou 2, **caractérisé en ce que** le au moins un premier membre de blocage (50, 51) et le au moins un second membre de blocage (55, 56) sont en appui l'un contre l'autre dans la position bloquée.

4. Implant selon l'une des revendications précédentes, **caractérisé en ce que** le second composant d'implant (14) comprend le au moins un second membre de blocage (55, 56).

5. Implant selon la revendication 4, **caractérisé en ce que** le second composant d'implant (14) présente un élément de fixation (32) coopérant avec l'élément de verrouillage (41) pour le verrouillage de l'implant (10) qui comprend le au moins un second membre de blocage (55, 56).

6. Implant selon l'une des revendications précédentes, **caractérisé en ce qu'**au moins un membre de blocage (50, 51, 55, 56) et/ou l'élément de verrouillage (41) et/ou un élément de fixation (32) coopérant avec l'élément de verrouillage (41) pour le verrouillage de l'implant (10) du second composant d'implant (14) sont agencés de manière rigide.

7. Implant selon l'une des revendications précédentes, **caractérisé en ce que** l'élément de verrouillage (41) et le composant d'implant (14) comprenant le au moins un second membre de blocage (55, 56) comprennent respectivement deux membres de blocage (50, 51, 55, 56) coopérant par paire dans la position bloquée.

8. Implant selon l'une des revendications précédentes, **caractérisé en ce que** l'élément de verrouillage (41) et le composant d'implant (14) comprenant le au moins un second membre de blocage (55, 56) s'accrochent l'un à l'autre dans la position bloquée.

9. Implant selon l'une des revendications précédentes, **caractérisé en ce que** l'élément de verrouillage (41) est agencé de manière déplaçable par rapport au premier composant d'implant (12) et/ou par rapport au second composant d'implant (14) pour le passage de la position de libération dans la position de verrouillage.

10. Implant selon l'une des revendications précédentes, **caractérisé en ce que** la direction de blocage est orientée perpendiculairement à la direction de verrouillage (46).

11. Implant selon l'une des revendications précédentes, **caractérisé en ce que** le second composant d'implant (14) est précontraint par rapport au premier composant d'implant (12) à l'encontre de la direction d'expansion (36) dans la position de verrouillage de l'élément de verrouillage (41).

12. Implant selon l'une des revendications précédentes, **caractérisé en ce que** la direction de blocage présente une composante parallèle à la direction d'expansion (36).

13. Système chirurgical comprenant au moins un implant (10) selon l'une des revendications précédentes ainsi qu'un dispositif de manipulation (71) pour le passage de l'élément de verrouillage (41) de la position de libération dans la position de verrouillage.

14. Système chirurgical selon la revendication 13, **caractérisé en ce que** le dispositif de manipulation (71) est prémonté sur l'implant (10).

15. Système chirurgical selon la revendication 13 ou 14, **caractérisé en ce que** le dispositif de manipulation (71) comprend un mécanisme de liaison (79) pour la liaison avec un applicateur comprenant un élément de préhension.
